# EUROPEAN PATENT APPLICATION

(11) **EP 2 360 752 A1**
(43) Date of publication of application: **24.08.2011**
(21) Application number: 09827568.8
(22) Date of filing: 18.11.2009
(51) Int. Cl.: H01L 51/50, C09K 11/06, C07D 471/04, C07F 15/00

(54) **ORGANIC ELECTROLUMINESCENT ELEMENT**

(30) Priority: 21.11.2008 JP 2008298212
(71) Applicant: FUJIFILM Corporation, Tokyo 106-8620 (JP)
(72) Inventor: SHIBATA, Kazuyuki, Ashigarakami-gun Kanagawa 258-8577 (JP); SOTOYAMA, Wataru, Ashigarakami-gun Kanagawa 258-8577 (JP)
(74) Representative: Siegert, Georg
(86) International application number: PCT/JP2009/069543
(87) International publication number: WO 2010/058787

(57) **Abstract**

An organic electroluminescent device, comprising a pair of electrodes and an organic compound layer disposed between the pair of electrodes, the organic compound layer comprising at least a luminescent layer, a first electron transporting layer provided so as to contact a cathode-side surface of the luminescent layer, and a second electron transporting layer provided so as to contact a cathode-side surface of the first electron transporting layer, wherein the luminescent layer comprises at least a luminescent material represented by following formula (1) and a hole transporting host material, and the second electron transporting layer comprises at least an electron transporting material represented by following formula (ET) and at least one selected from the group consisting of alkali metals, alkali metal salts, alkaline earth metals and alkaline earth metal salts.

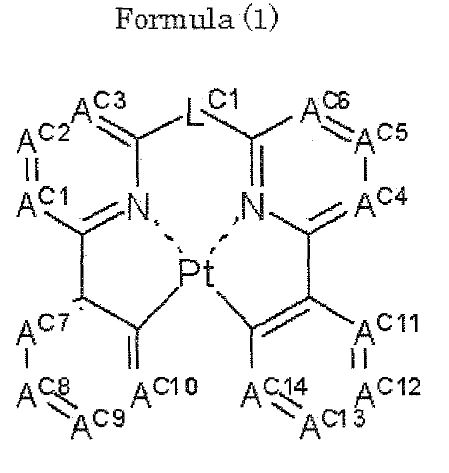

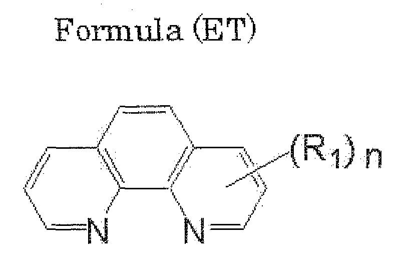

## Description

### Technical Field

The invention relates to an organic electroluminescent device, which may be referred to as an organic EL device hereinafter, that is effectively usable for a flat light source, such as a full color display, a backlight or an illumination light source, for a light source array in a printer or the like, or for some other.

### Background Art

The organic EL device is composed of a luminescent layer or plural organic compound layers containing a luminescent layer, and opposite electrodes between which the organic compound layers are disposed. The organic EL device is a luminescence-gaining device making use of at least one of luminescence from excitons generated by recombination of electrons injected from its cathode with holes injected from its anode in the organic compound layers, and luminescence from excitons of a different molecule that are generated by energy shift from the above-mentioned excitons.

Hitherto, about organic EL devices, a structure of laminated layers which have functions separated from each other, respectively, has been used, whereby the devices have been greatly improved in luminance or device efficiency, so as to be developed. For example, the following devices have been frequently used: a bilayered lamination type device, wherein a hole transporting layer and an electron transporting layer which functions also as a luminescent layer are laminated; a three-layer lamination type device, wherein a hole transporting layer, a luminescent layer, and an electron transporting layer are laminated; and a four-layer lamination type device, wherein a hole transporting layer, a luminescent layer, a hole blocking layer, and an electron transporting layer are laminated.

However, in order to put organic EL devices into practical use, there have still remained many themes, for example, an enhancement in their luminescence efficiency, and an improvement in their driving endurance. In particular, the enhancement in the luminescence efficiency makes it possible to decrease the consumption of electric power, and further gives advantages for the driving endurance; hitherto, therefore, many improving manners have been disclosed. However, it is not easy to recombine electrons injected from a cathode effectively with holes injected from the (concerned) anode in the luminescent layer. Thus, there may be an inefficient case in which the recombination is not caused so that holes or electrons leak toward the cathode or anode. This hole- or electron-leakage, which does not contribute to the recombination, causes not only a reduction in the efficiency but also a problem that the leaking holes or electrons produce an effect onto the electron transporting layer or the hole transporting layer to deteriorate the driving endurance. Disclosed is, for example, a structure in which a hole blocking layer doped with an electron donating metal complex, such as BAlq or Alq, is provided on the surface of a luminescent layer which faces a cathode, and it is stated that this structure gives highly bright luminescence at a low driving voltage (see, for example, Patent Document 1). In the case of using an iridium complex as a luminescent material, a structure is disclosed which has an electron transporting layer, and a mixed layer arranged between the electron transporting layer and a cathode, the mixed layer containing an electron transporting material of the electron transporting layer, and an electron donating material, and it is stated that this structure gives an improved luminescence efficiency (see, for example, Patent Document 2).
Patent Document 1: Japanese Patent Laid-Open (JP-A) No. 2007-96066
Patent Document 2: JP-A No. 2006-352102

### Disclosure of the Invention

### Technical Problem

An object of the invention is to provide an organic EL device high in luminescence efficiency, low in driving voltage, and excellent in driving endurance.

### Solution to Problem

In light of the above-mentioned problems, the invention has been made, and the object is attained by the following subject matters:
<1> An organic electroluminescent device, comprising a pair of electrodes and an organic compound layer disposed between the pair of electrodes, the organic compound layer comprising at least a luminescent layer, a first electron transporting layer provided so as to contact a cathode-side surface of the luminescent layer, and a second electron transporting layer provided so as to contact a cathode-side surface of the first electron transporting layer, wherein the luminescent layer comprises at least a luminescent material represented by following formula (1) and a hole transporting host material, and the second electron transporting layer comprises at least an electron transporting material represented by following formula (ET) and at least one selected from the group consisting of alkali metals, alkali metal salts, alkaline earth metals and alkaline earth metal salts:

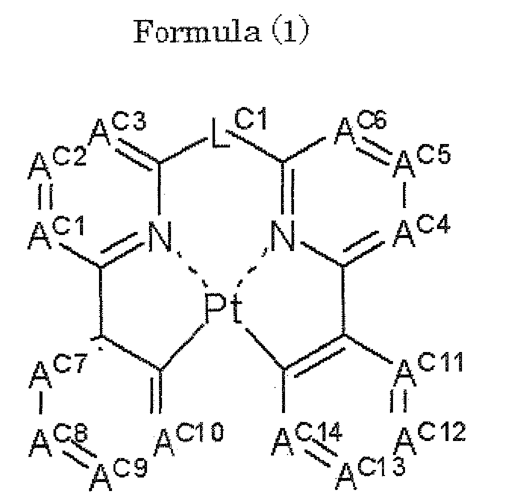

wherein, in formula (1), A^{C1} to A^{C14} each independently represents C-R or N, wherein R represents a hydrogen atom or a substituent, and L^{C1} represents a single bond or a bivalent linking group; and

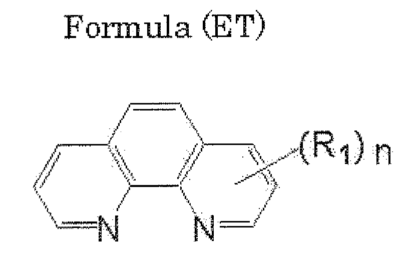

wherein, in formula (ET), R₁ each represents a hydrogen atom, or a substituent selected from the group consisting of alkyl groups having 1 to 10 carbon atoms and substituted or unsubstituted aryl groups having 6 to 30 carbon atoms; and n represents an integer of 0 to 8 provided that when n is an integer of 2 or more, the R₁'s may be the same as or different from each other.

<2> The organic electroluminescent device of item <1>, wherein the compound represented by the formula (1) is a compound represented by following formula (2):

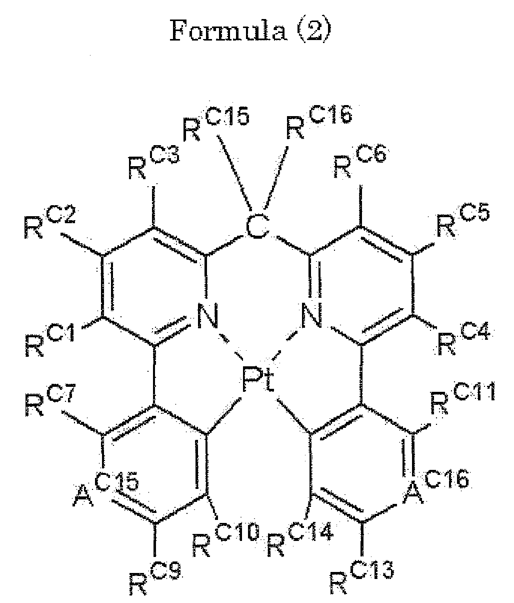

wherein, in formula (2), A^{C15} and A^{C16} each independently represents C-R or N, wherein R represents a hydrogen atom or a substituent; and R^{C1} to R^{C16} each represent a hydrogen atom or a substituent.

<3> The organic electroluminescent device of item <1> or <2>, wherein the at least one selected from the group consisting of the alkali metals, alkali metal salts, alkaline earth metals, and alkaline earth metal salts is selected from Li, Cs, Ca, LiF, NaF, KF, RbF, CsF, MgF₂, CaF₂, SrF₂, BaF₂, LiCl, NaCl, KCl, RbCl, CsCl, MgCl₂, CaCl₂. SrCl₂, BaCl₂, LiHCO₃, NaHCO₃, KHCO₃, RbHCO₃, CsHCO₃, Li₂CO₃, Na₂CO₃, K₂CO₃, Rb₂CO₃, Cs₂CO₃, MgCO_{3,} CaCO₃, SrCO₃, and BaCO₃.

<4> The organic electroluminescent device of any one of items <1> to <3>, wherein in the second electron transporting layer, a ratio by mass of a total of the selected alkali metal(s), alkali metal salt(s), alkaline earth metal(s) and alkaline earth metal salt(s) in the electron transporting material represented by formula (ET) is from 0.1 to 2.0% by mass.
<5> The organic electroluminescent device of any one of items <1> to <4>, wherein the hole transporting host material is a carbazole derivative.

### Advantageous Effects of Invention

According to the invention, provided is an organic EL device high in luminescence efficiency, low in driving voltage, and excellent in driving endurance.

### Best Mode for Carrying out the Invention

Hereinafter, the organic electroluminescent device of the invention is described in detail.
The organic EL device of the invention is an organic electroluminescent device, comprising a pair of electrodes and an organic compound layer disposed between the pair of electrodes, the organic compound layer comprising at least a luminescent layer, a first electron transporting layer provided so as to contact a cathode-side surface of the luminescent layer, and a second electron transporting layer provided so as to contact a cathode-side surface of the first electron transporting layer, wherein the luminescent layer comprises at least a luminescent material represented by following formula (1) and a hole transporting host material, and the second electron transporting layer comprises at least an electron transporting material represented by following formula (ET) and at least one selected from the group consisting of alkali metals, alkali metal salts, alkaline earth metals and alkaline earth metal salts:

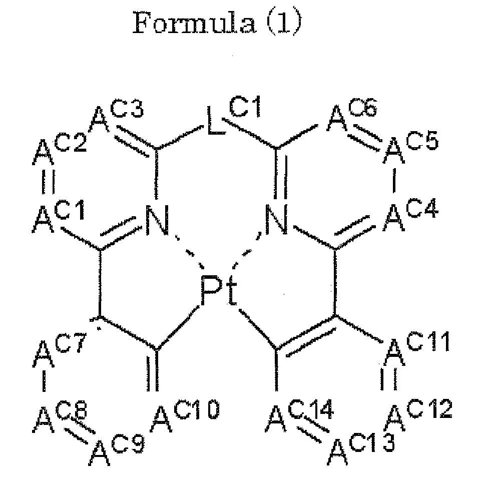

In formula (1), A^{C1} to A^{C14} each independently represents C-R or N, wherein R represents a hydrogen atom or a substituent; and L^{C1} represents a single bond or a bivalent linking group.

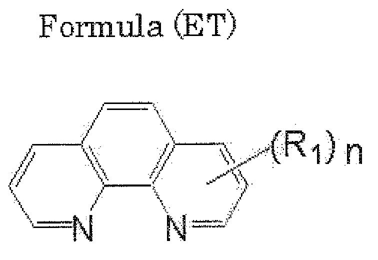

In formula (ET), R₁ each represents a hydrogen atom, or a substituent selected from the group consisting of alkyl groups having 1 to 10 carbon atoms and substituted or unsubstituted aryl groups having 6 to 30 carbon atoms; and n represents an integer of 0 to 8 provided that when n is an integer of 2 or more, the R₁'s may be the same as or different from each other.

In light of the nature of the luminescent device, at least one electrode of its anode and its cathode is preferably transparent or translucent.

A preferred embodiment of the organic compound layer in the invention is an embodiment wherein from the anode side, a hole transporting layer, the luminescent layer, the first electron transporting layer, and the second electron transporting layer are laminated in this order. The organic compound layer may have a charge blocking layer or some other layer between the hole transporting layer and the luminescent layer or between the luminescent layer and the electron transporting layers. The compound layer may have a hole injecting layer between the anode and the hole transporting layer, and may have an electron injecting layer between the cathode and the electron transporting layers. Each of the layers may be divided into plural secondary layers.

1. Description of the Compound Represented by formula (1)
Next, a detailed description is made about the compound represented by formula (1), which is used in the organic electroluminescent device of the invention.

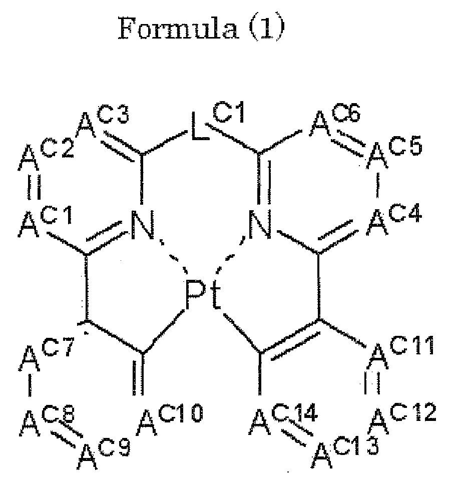

In formula (1), A^{C1} to A^{C14} each independently represent C-R or N. C-R denotes that a carbon atom is an atom which constitutes the concerned ring, and R represents a hydrogen atom or a substituent on the carbon atom. A^{C1} to A^{C6} each independently represent C-R or N wherein R represents a hydrogen atom or a substituent. Examples of the substituent represented by R include alkyl groups (having preferably 1 to 30 carbon atoms, more preferably 1 to 20 carbon atoms, in particular preferably 1 to 10 carbon atoms, such as methyl, ethyl, iso-propyl, tert-butyl, n-octyl, n-decyl, n-hexadecyl, cyclopropyl, cyclopentyl, and cyclohexyl), alkenyl groups (having preferably 2 to 30 carbon atoms, more preferably 2 to 20 carbon atoms, in particular preferably 2 to 10 carbon atoms, such as vinyl, allyl, 2-butenyl, and 3-pentenyl), alkynyl groups (having preferably 2 to 30 carbon atoms, more preferably 2 to 20 carbon atoms, in particular preferably 2 to 10 carbon atoms, such as propargyl, and 3-pentynyl), aryl groups (having preferably 6 to 30 carbon atoms, more preferably 6 to 20 carbon atoms, in particular preferably 6 to 12 carbon atoms, such as phenyl, p-methylphenyl, naphthyl, and anthranyl), amino groups (having preferably 0 to 30 carbon atoms, more preferably 0 to 20 carbon atoms, in particular preferably 0 to 10 carbon atoms, such as amino, methylamino, dimethylamino, diethylamino, dibenzylamino, diphenylamino, and ditolylamino), alkoxy groups (having preferably 1 to 30 carbon atoms, more preferably 1 to 20 carbon atoms, in particular preferably 1 to 10 carbon atoms, such as methoxy, ethoxy, butoxy, and 2-ethylhexyloxy), and aryloxy groups (having preferably 6 to 30 carbon atoms, more preferably 6 to 20 carbon atoms, in particular preferably 6 to 12 carbon atoms, such as phenyloxy, 1-naphthyloxy, and 2-naphthyloxy);

heterocyclic oxy groups (having preferably 1 to 30 carbon atoms, more preferably 1 to 20 carbon atoms, in particular preferably 1 to 12 carbon atoms, such as pyridyloxy, pyrazyloxy, pyrimidyloxy, and quinolyloxy), acyl groups (having preferably I to 30 carbon atoms, more preferably 1 to 20 carbon atoms, in particular preferably 1 to 12 carbon atoms, such as acetyl, benzoyl, formyl, and pivaloyl), alkoxycarbonyl groups (having preferably 2 to 30 carbon atoms, more preferably 2 to 20 carbon atoms, in particular preferably 2 to 12 carbon atoms, such as methoxycarbonyl, and ethoxycarbonyl), aryloxycarbonyl groups (having preferably 7 to 30 carbon atoms, more preferably 7 to 20 carbon atoms, in particular preferably 7 to 12 carbon atoms, such as phenyloxycarbonyl), acyloxy groups (having preferably 2 to 30 carbon atoms, more preferably 2 to 20 carbon atoms, in particular preferably 2 to 10 carbon atoms, such as acetoxy, and benzoyloxy), acylamino groups (having preferably 2 to 30 carbon atoms, more preferably 2 to 20 carbon atoms, in particular preferably 2 to 10 carbon atoms, such as acetylamino, and benzoylamino), alkoxycarbonylamino groups (having preferably 2 to 30 carbon atoms, more preferably 2 to 20 carbon atoms, in particular preferably 2 to 12 carbon atoms, such as methoxycarbonylamino), aryloxycarbonylamino groups (having preferably 7 to 30 carbon atoms, more preferably 7 to 20 carbon atoms, in particular preferably 7 to 12 carbon atoms, such as phenyloxycarbonylamino), and sulfonylamino groups (having preferably 1 to 30 carbon atoms, more preferably 1 to 20 carbon atoms, in particular preferably 1 to 12 carbon atoms, such as methanesulfonylamino, and benzenesulfonyl amino);

sulfamoyl groups (having preferably 0 to 30 carbon atoms, more preferably 0 to 20 carbon atoms, in particular preferably 0 to 12 carbon atoms, such as sulfamoyl, methylsulfamoyl, dimethylsulfamoyl, and phenylsulfamoyl), carbamoyl groups (having preferably 1 to 30 carbon atoms, more preferably 1 to 20 carbon atoms, in particular preferably 1 to 12 carbon atoms, such as carbamoyl, methylcarbamoyl, diethylcarbamoyl and phenylcarbamoyl), alkylthio groups (having preferably 1 to 30 carbon atoms, more preferably 1 to 20 carbon atoms, in particular preferably 1 to 12 carbon atoms, such as methylthio, and ethylthio), arylthio groups (having preferably 6 to 30 carbon atoms, more preferably 6 to 20 carbon atoms, in particular preferably 6 to 12 carbon atoms, such as phenylthio), heterocyclic thio groups (having preferably 1 to 30 carbon atoms, more preferably 1 to 20 carbon atoms, in particular preferably 1 to 12 carbon atoms, such as pyridylthio, 2-benzimidazolylthio, 2-benzoxazolylthio, and 2-benzthiazolylthio), sulfonyl groups (having preferably 1 to 30 carbon atoms, more preferably 1 to 20 carbon atoms, in particular preferably I to 12 carbon atoms, such as mesyl, and tosyl), and sulfinyl groups (having preferably 1 to 30 carbon atoms, more preferably 1 to 20 carbon atoms, in particular preferably 1 to 12 carbon atoms, such as methanesulfinyl, and benzenesulfinyl);

ureido groups (having preferably 1 to 30 carbon atoms, more preferably 1 to 20 carbon atoms, in particular preferably 1 to 12 carbon atoms, such as ureido, methylureido, and phenylureido), phosphoric amide groups (having preferably 1 to 30 carbon atoms, more preferably 1 to 20 carbon atoms, in particular preferably 1 to 12 carbon atoms, such as diethylphosphoric amide, and phenylphosphoric amide), a hydroxy group, a mercapto group, halogen atoms (such as fluorine, chlorine, bromine, and iodine atoms), a cyano group, a sulfo group, a carboxyl group, a nitro group, a hydroxamic acid group, a sulfino group, a hydrazino group, an imino group, heterocyclic groups (having preferably 1 to 30 carbon atoms, more preferably 1 to 12 carbon atoms, and containing, as the heteroatom(s), a nitrogen atom, an oxygen atom, and/or a sulfur atom; specific examples of the heterocyclic groups including imidazolyl, pyridyl, xynolyl, furyl, thienyl, piperidyl, morpholino, benzoxazolyl, benzimidazolyl, benzthiazolyl, carbazolyl, and azepinyl groups), silyl groups (having preferably 3 to 40 carbon atoms, more preferably 3 to 30 carbon atoms, in particular preferably 3 to 24 carbon atoms, such as trimethylsilyl, and triphenylsilyl), and silyloxy groups (having preferably 3 to 40 carbon atoms, more preferably 3 to 30 carbon atoms, in particular preferably 3 to 24 carbon atoms, such as trimethylsilyloxy, and triphenylsilyloxy). These substituents may each be further substituted.

R is preferably a hydrogen atom, an alky, aryl, cyano or amino group, or a fluorine radical. R is more preferably a hydrogen atom, or a fluorine radical.
A^{C1} to A^{C6} are each preferably C-R.

A^{C1} to A^{C6} are each preferably C-R. R's (of A^{C1} to A^{C6}) may be linked to each other to form a ring. When A^{C1} to A^{C6} are each C-R, R's in A^{C2} and A^{C5} are each preferably a hydrogen atom, an alkyl, aryl, amino, alkoxy, aryloxy or cyano group, or a fluorine radical, more preferably a hydrogen atom, an amino, alkoxy or aryloxy group, or a fluorine radical, in particular preferably a hydrogen atom, or a fluorine radical, and R's in A^{C1}, A^{C3}, A^{C4} and A^{C6} are each preferably a hydrogen atom, an alkyl, aryl, amino, alkoxy, aryloxy or cyano group, or a fluorine radical, more preferably a hydrogen atom, an amino, alkoxy or aryloxy group, or a fluorine radical, in particular preferably a hydrogen atom.

About A^{C1} to A^{C14}, in each of the moiety of A^{C7} to A^{C10} and that of A^{C11} to AC¹⁴, the number of Ns (representing nitrogen atoms) is preferably from 0 to 2, more preferably from 0 to 1. It is preferred that one or more symbols selected from A^{C8} to A^{C10} (each) represent(s) N, and one or more symbols selected from A^{C12} to A^{C14} is/are (each) N. It is more preferred that one or more symbols selected from A^{C8}, A^{C9}, A^{C12}, and A^{C13} (each) represent(s) N, and it is particularly preferred that one or two symbols selected from A^{C8} and A^{C12} (each) represent(s) N.

When A^{C7} to A^{C14} each represent C-R, R of each of A^{C8} and A^{C12} is preferably a hydrogen atom, an alkyl, fluoroalkyl, aryl, amino, alkoxy, aryloxy or cyano group, or a fluorine radical, more preferably a hydrogen atom, a fluoroalkyl, alkyl, aryl or cyano group, or a fluorine radical, in particular preferably a hydrogen atom, or a polyfluoroalkyl or cyano group. R represented by A^{C7}, A^{C9}, A^{C11} or A^{C13} is preferably a hydrogen atom, an alkyl, fluoroalkyl, aryl, amino, alkoxy, aryloxy or cyano group, or a fluorine radical, more preferably a hydrogen atom, a fluoroalkyl or cyano group, or a fluorine radical, in particular preferably a hydrogen atom, a fluorine radical.

R represented by each of A^{C10} and AC¹⁴ is preferably a hydrogen atom or a fluorine radical, more preferably a hydrogen atom. When any two or more of A^{C7} to A^{C9} and A^{C11} to A^{C13} each represent C-R, Rs may be linked to each other to form a ring. L^{C1} represents a single bond or a divalent linking group. Examples of the divalent linking group represented by L^{C1} include alkylene groups (such as methylene, ethylene, and propylene), arylene groups (such as phenylene, and naphthalenediyl), hetero arylene groups (such as pyridinediyl, and thiophenediyl), imino groups (-NR-) (such as a phenylimino group), an oxy group (-O-), a thio group (-S-), phosphinidene groups (-PR-) (such as a phenylphosphinidene group), silylene groups (-SiRR'-) (such as dimethylsilylene and diphenylsilylene groups), and combinations of two or more thereof. These linking groups may each have a substituent. L^{C1} is preferably a single bond, or an alkylene, arylene, hetero arylene, amino, oxy, thio or silylene group, more preferably a single bond, or an alkylene, arylene or amino group, even more preferably an alkylene group, even more preferably a methylene group, even more preferably a di-substituted methylene group, even more preferably a dimethytmethylene, diethylmethylene, diisobutylmethylene, dibenzylmethylene, ethylmethylmethylene, methylpropylmethylene, isobutylmethylmethylene, diphenylmethylene, methylphenylmethylene, cyclohexanediyl, cyclopentanediyl, fluorenediyl or fluoromethylmethylene group, in particular preferably a dimethylmethylene, diphenylmethylene or cyclohexanediyl group.

Of compounds represented by formula (1), a compound represented by the following formula (2) is preferred:

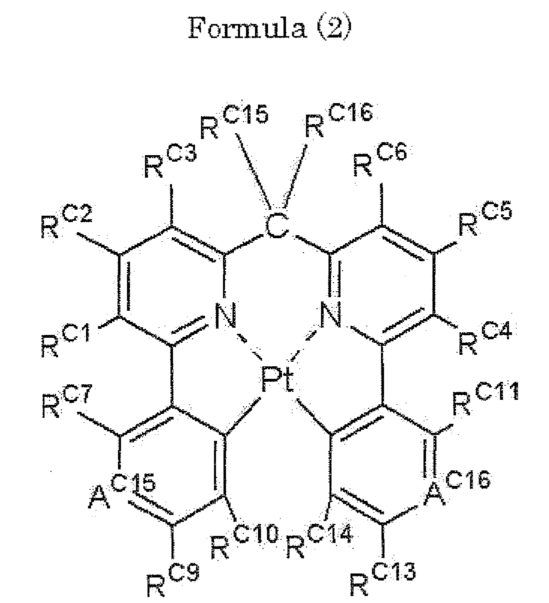

In formula (2), A^{C15} and A^{C16} each independently represent C-R or N. C-R denotes that a carbon atom is an atom which constitutes the concerned ring and R is a hydrogen atom or a substituent on the carbon atom. R^{C1} to R^{C16} each represent a hydrogen atom or a substituent.

A description is made about formula (2). A^{C15} and A^{C16} each independently represent C-R or N. When A^{C15} or A^{C16} represents C-R, R of A^{C15} or A^{C16} is preferably a hydrogen atom, an alkyl, fluoroalkyl, aryl, amino, alkoxy, aryloxy or cyano group, or a fluorine radical, more preferably a hydrogen atoms, a fluoroalkyl, alkyl, aryl or cyano group, or a fluorine radical, in particular preferably a hydrogen atom, or a fluoroalkyl or cyano group. R^{C1} to R^{C16} each represent a hydrogen atom or a substituent. R^{C1} to R^{C16} are each preferably a hydrogen atom, an alkyl, aryl, amino, alkoxy, aryloxy or cyano group, or a fluorine radical, more preferably a hydrogen atom, an amino, alkoxy or aryloxy group, or a fluorine radical, in particular preferably a hydrogen atom, or a fluorine radical, especially preferably a hydrogen atom. R^{C7} , R^{C9}, R^{C11} and R^{C13} are each preferably a hydrogen atom, an alkyl, fluoroalkyl, aryl, amino, alkoxy, aryloxy or cyano group, or a fluorine radical, more preferably a hydrogen atom, a fluoroalkyl or cyano group, or a fluorine radical, in particular preferably a hydrogen atom, or fluorine radical. R^{C10} and R^{C14} are each preferably a hydrogen atom, or a fluorine radical, more preferably a hydrogen atom. R^{C15} and R^{C16} are each preferably a hydrogen atom, a methyl, ethyl, propyl, butyl, fluoromethyl, isobutyl, benzyl or phenyl group, or a fluorine radical; or are preferably groups in which R^{C15} and R^{C16} are linked to each other to form a cyclohexane ring, groups in which R^{C15} and R^{C16} are linked to each other to form a cyclopentane ring, or groups in which R^{C15} and R^{C16} are linked to each other to form a fluorene ring. R^{C15} and R^{C16} are each more preferably a methyl, ethyl, isobutyl, benzyl or phenyl group; or are more preferably groups in which R^{C15} and R^{C16} are linked to each other to form a cyclohexane ring, groups in which R^{C15} and R^{C16} are linked to each other to form a cyclopentane ring, or groups in which R^{C15} and R^{C16} are linked to each other to form a fluorene ring. R^{C15} and R^{C16} are each even more preferably a methyl, ethyl, isobutyl, phenyl group; or are even more preferably groups in which R^{C15} and R^{C16} are linked to each other to form a cyclohexane ring. R^{C15} and R^{C16} are each in particular preferably a methyl or phenyl group.

Specific examples of the compounds represented by formulae (1) and (2), respectively, are listed up below. In the invention, however, the compounds are not limited thereto.

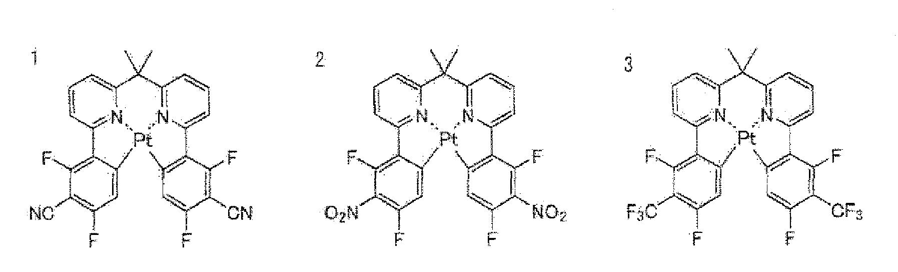

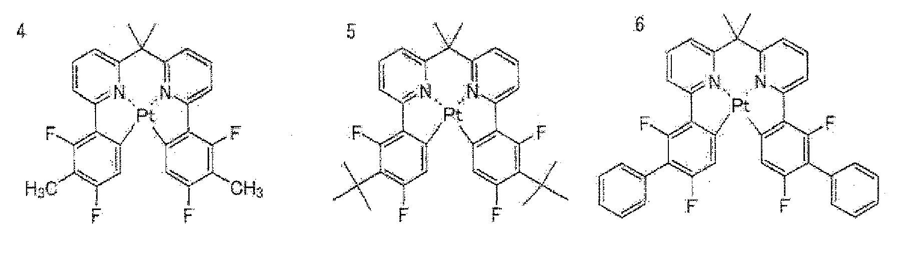

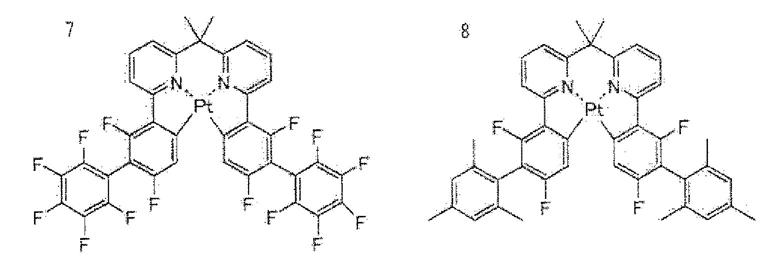

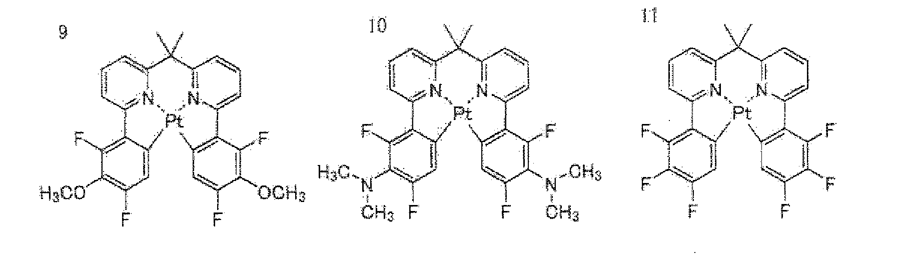

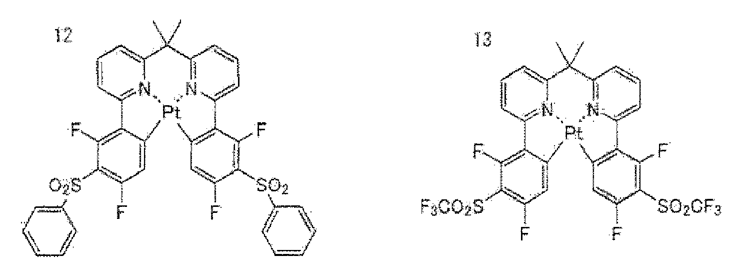

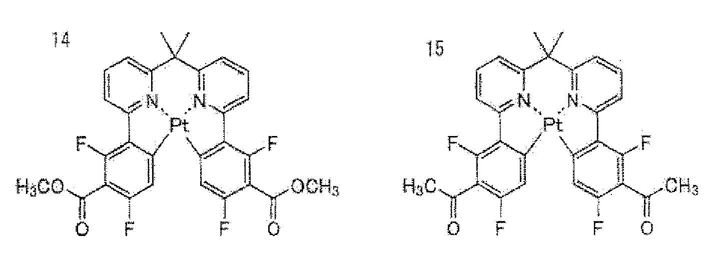

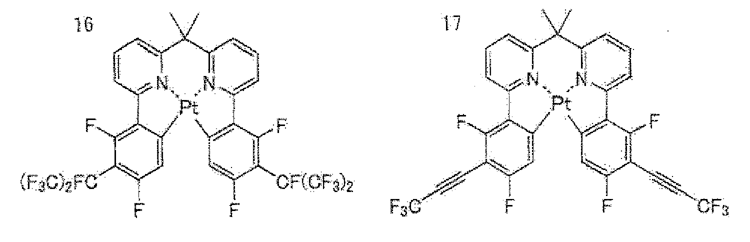

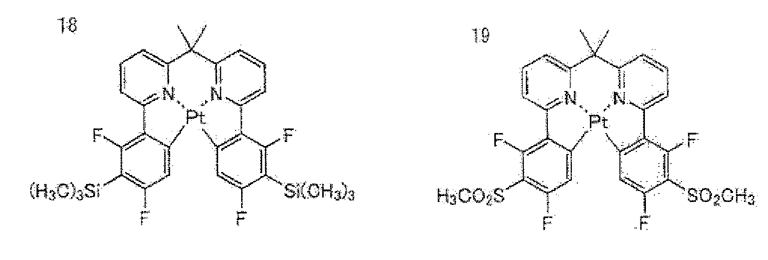

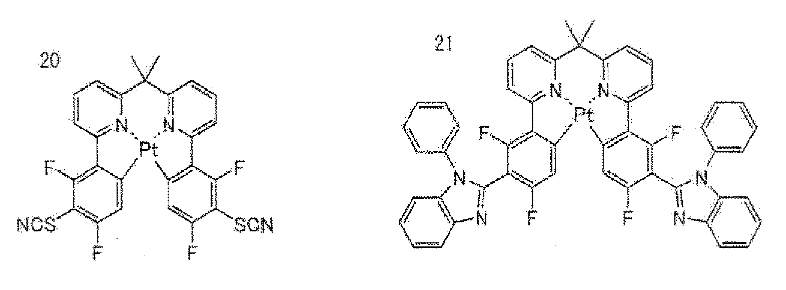

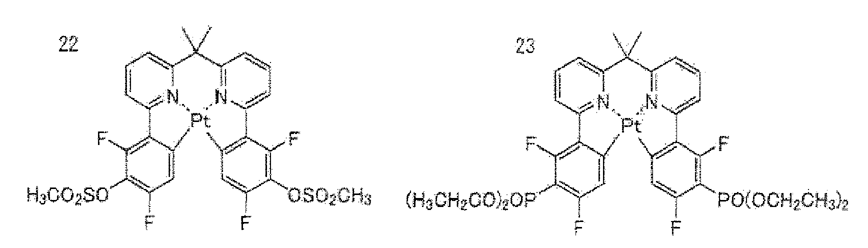

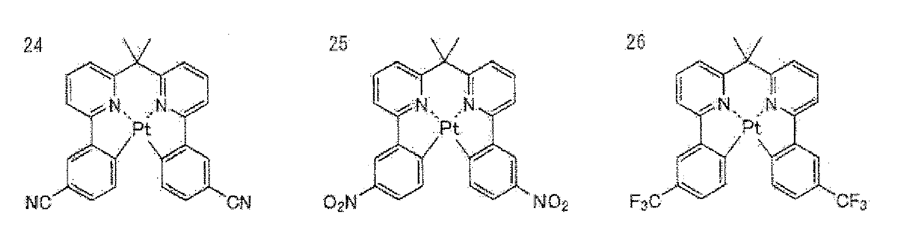

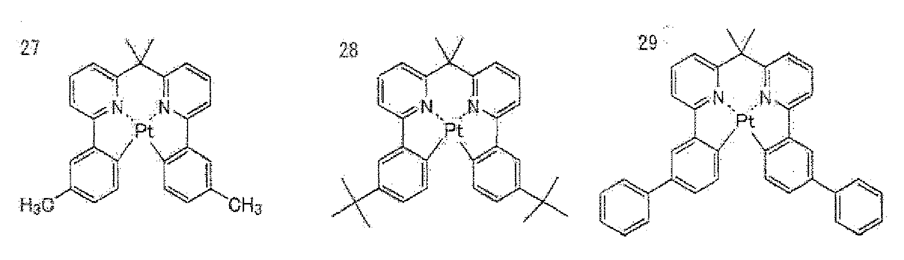

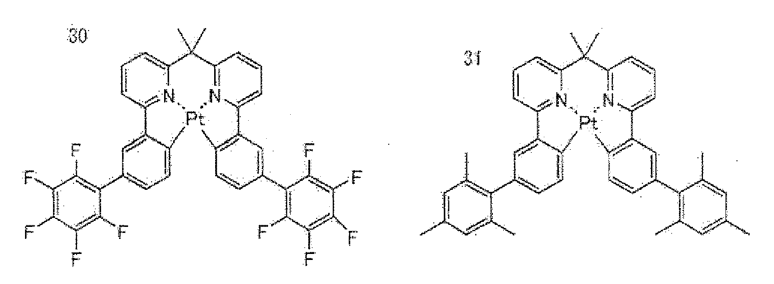

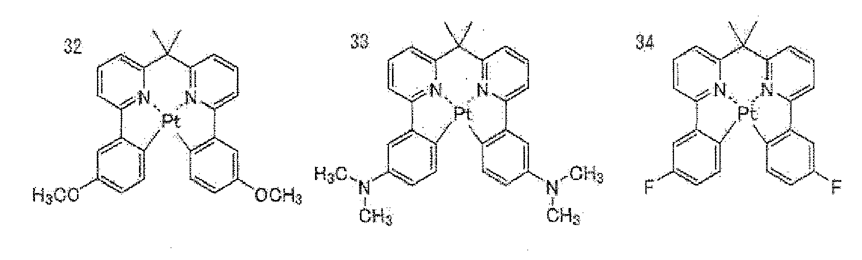

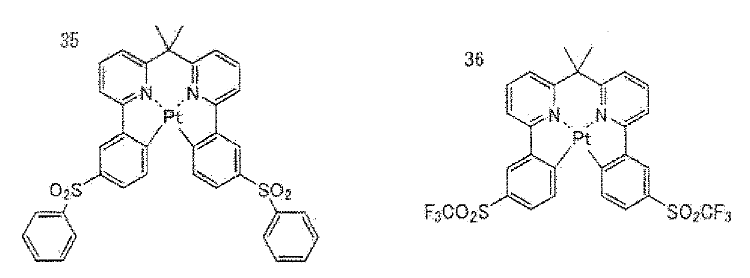

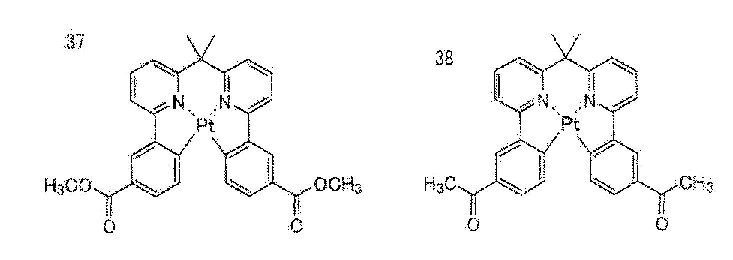

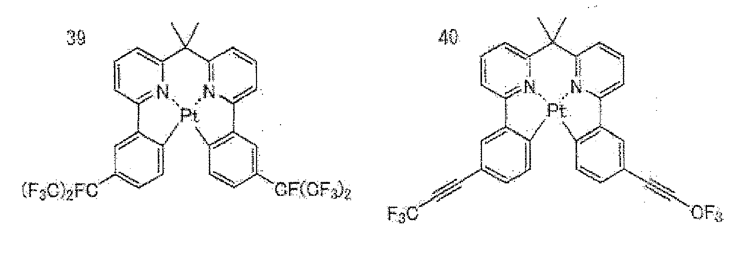

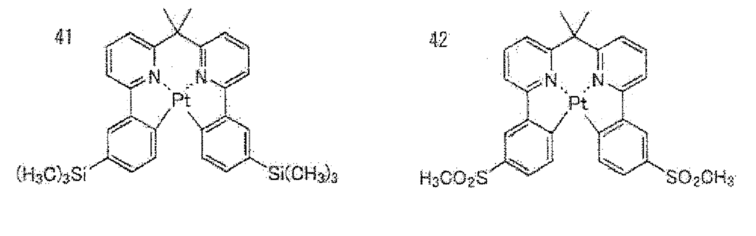

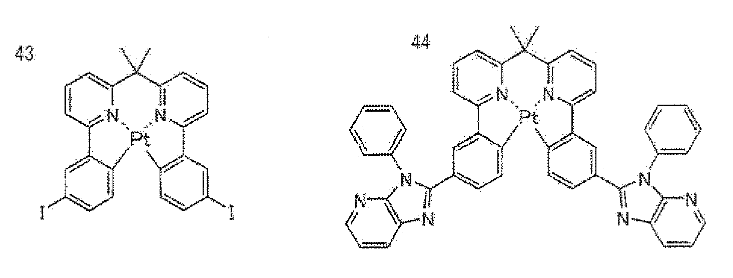

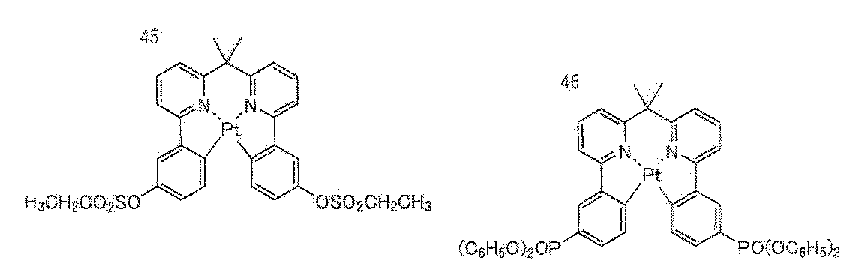

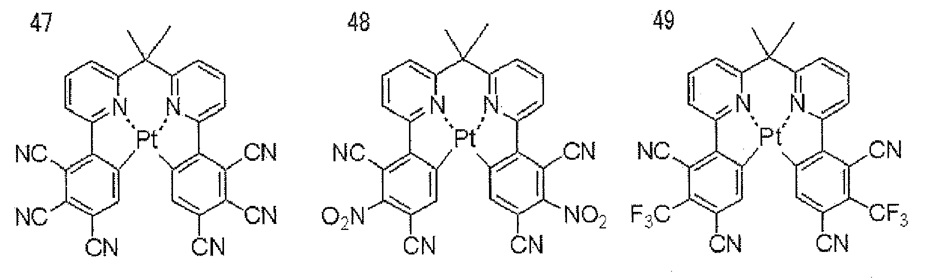

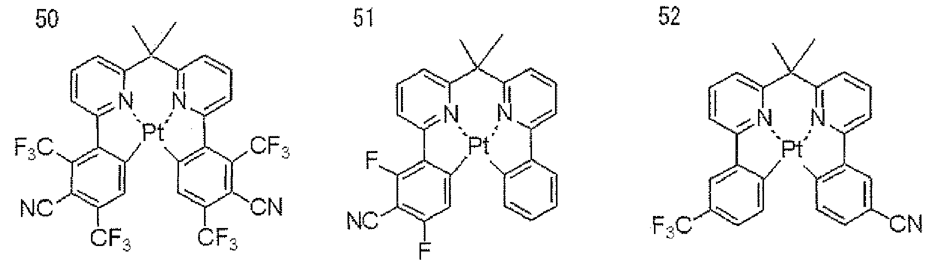

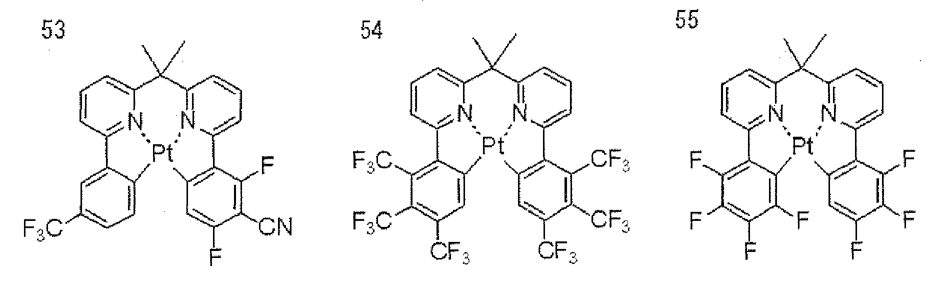

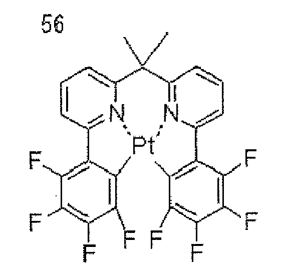

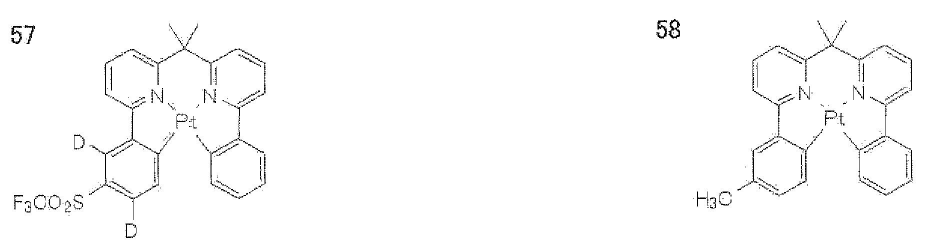

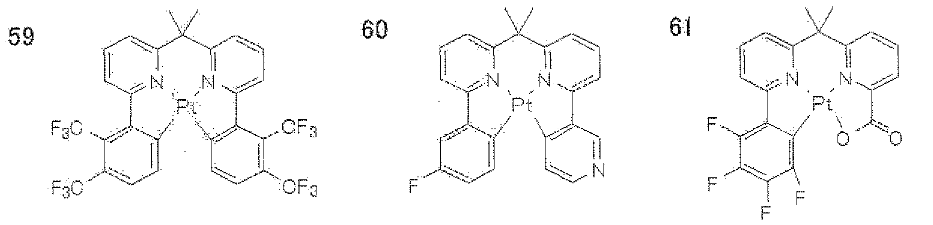

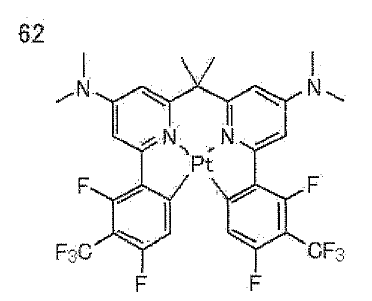

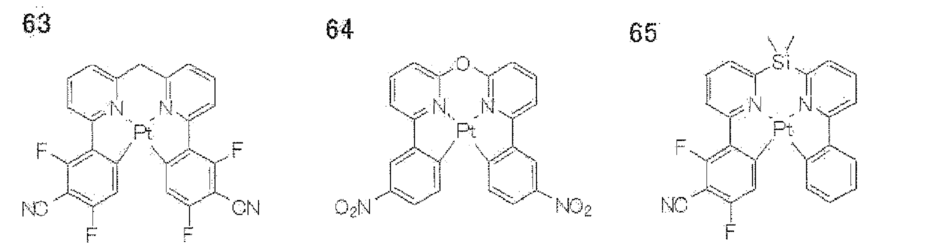

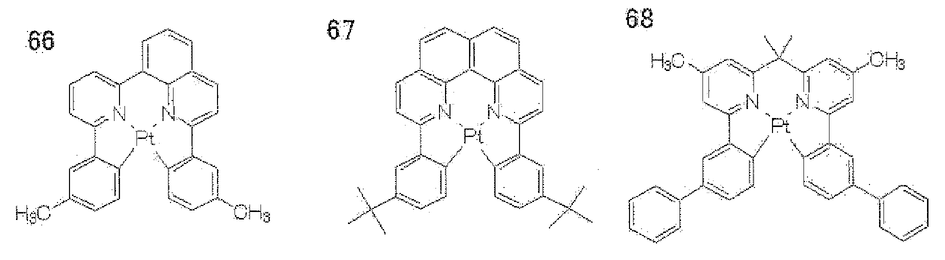

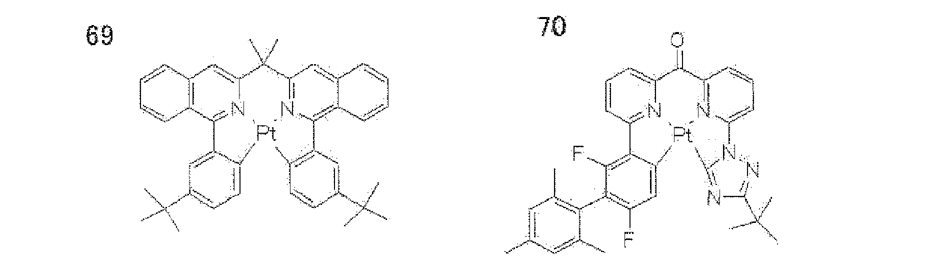

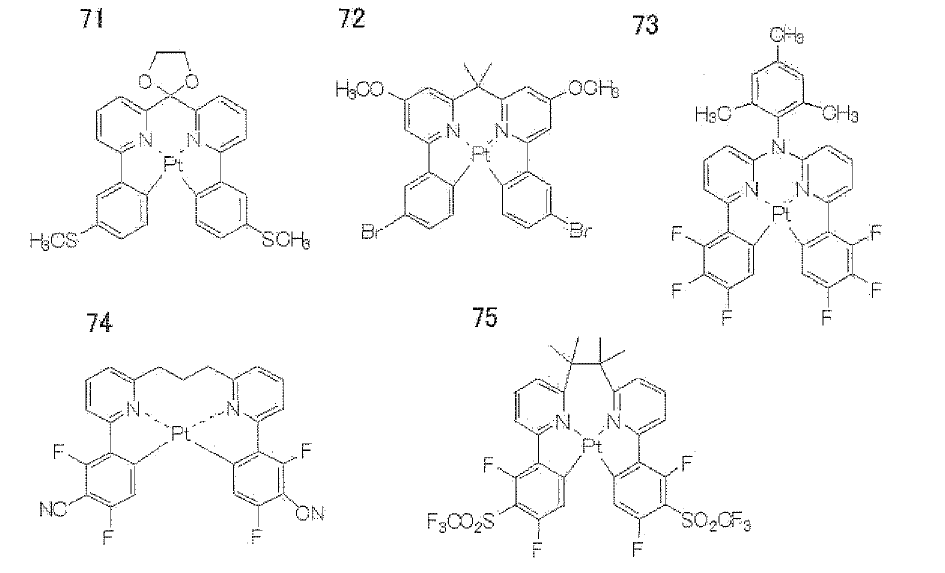

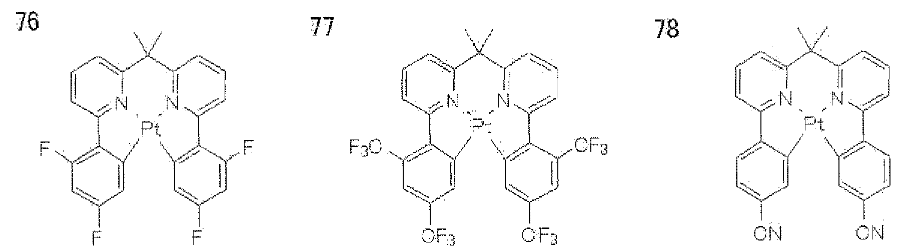

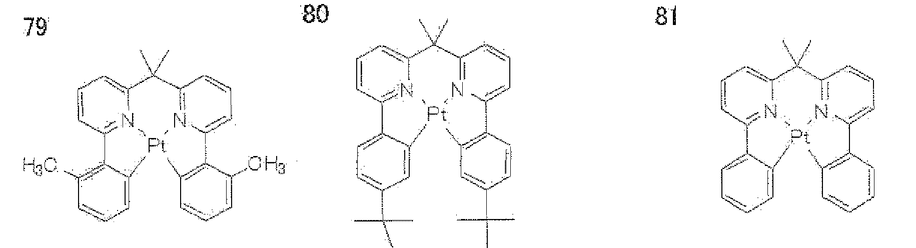

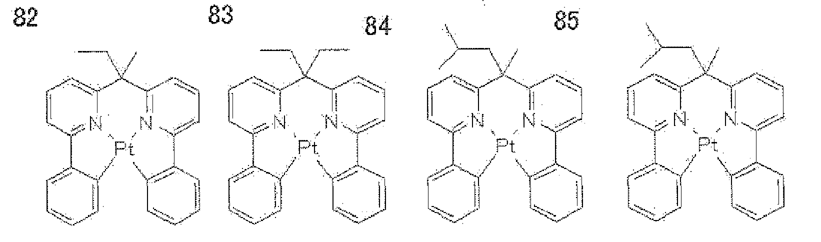

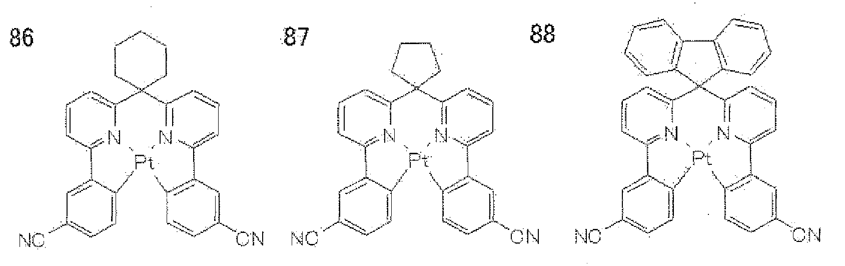

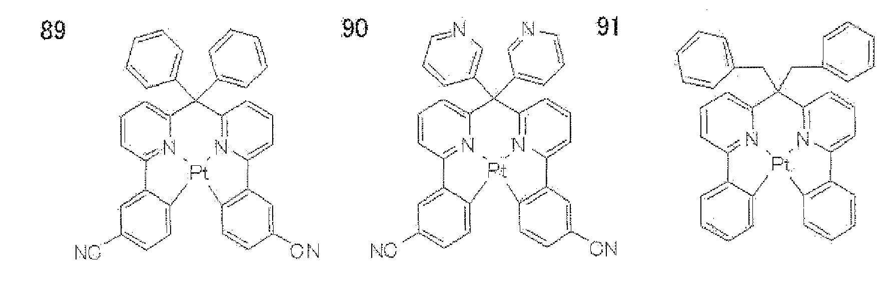

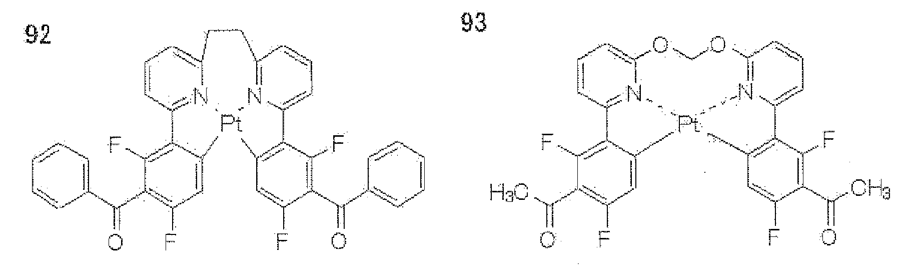

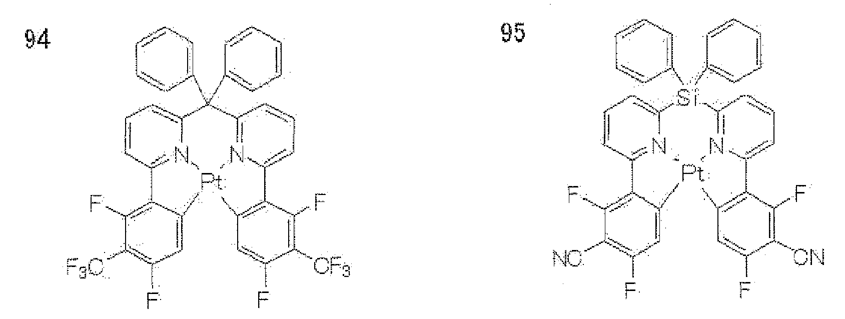

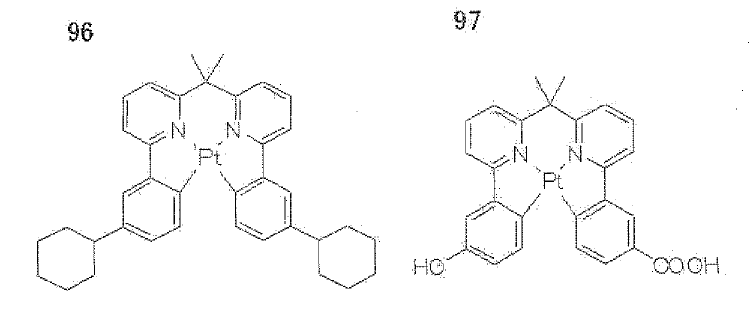

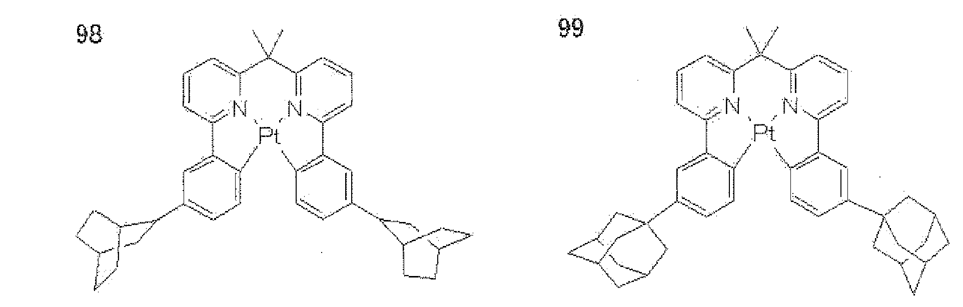

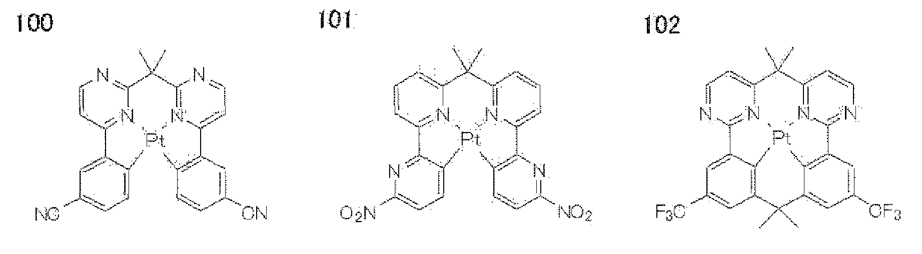

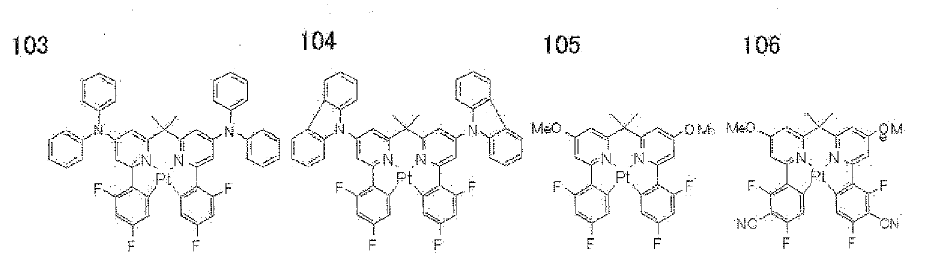

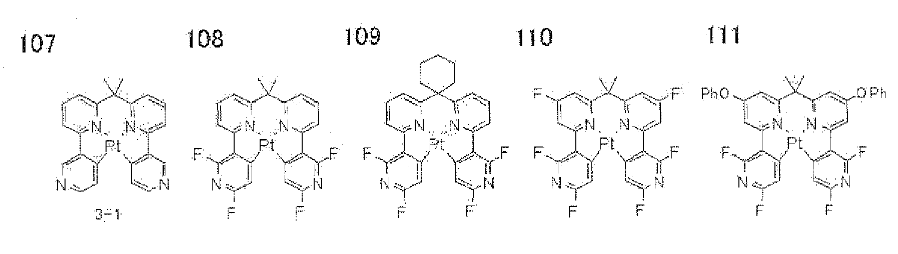

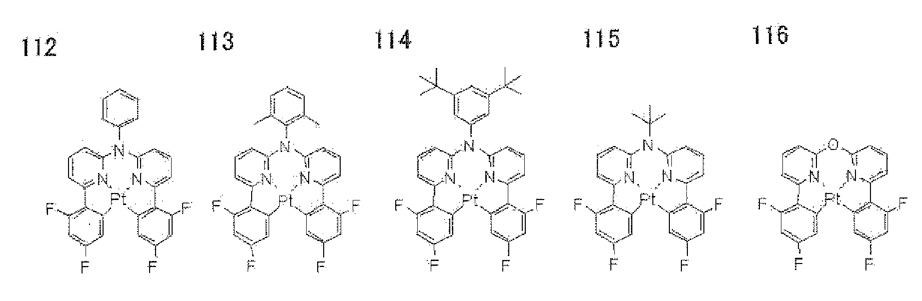

In the invention, compounds represented by formula (1) or (2) may be used alone or in the form of a mixture of two or more thereof.
When compounds represented by formula (1) are used in the form of a mixture in the invention, the blend ratio by mass therebetween is preferably from 0.1/99.9 to 99.9/0.1, more preferably from 1/99 to 99/1.

2. Luminescent Layer
The luminescent layer is a layer having a function of accepting holes from the anode, the hole injecting layer or the hole transporting layer at the time of applying an electric field thereto, and accepting electrons from the cathode, the electron injecting layer or the electron transporting layer at the same time to provide a site where the holes are recombined with the electrons, thereby generating luminescence.
The luminescent layer in the invention contains, as a luminescent material, a compound represented by formula (1) or (2), and contains, as a host material, a hole transporting host material. The luminescent layer in the invention may contain, besides these materials, a different phosphorescence emitting material or fluorescence emitting material as a luminescent material, or may contain, as a host material, an electron transporting host material.

In the invention, the thickness of the luminescent layer is not particularly limited, and is usually from 1 to 50 nm, preferably from 2 to 200 nm, more preferably from 3 to 100 nm.

2-1. Luminescent Material
The luminescent material is a compound represented by formula (1) or (2), which may be used together with a different fluorescence emitting material or phosphorescence emitting material. In order to promote the shift of energy from the host material, use may be made of a method disclosed in JP-A No. 2007-290748 or 2008-089843.

(a) Phosphorescence Emitting Material
The phosphorescence emitting material may be generally a metal complex containing a transition metal atom or a lanthanoid atom.
The transition metal atom is preferably ruthenium, rhodium, palladium, tungsten, rhenium, osmium, iridium, or platinum, more preferably rhenium, indium or platinum, even more preferably iridium or platinum.
Examples of the lanthanoid atom include lanthanum, cerium, praseodymium, neodymium, samarium, europium, gadolinium, terbium, dysprosium, holmium, erbium, thulium, ytterbium, and lutetium. Of these lanthanoid atoms, neodymium, europium and gadolinium are preferred.

Examples of a ligand of the complex include ligands described in G. Wilkinson et al., "Comprehensive Coordination Chemistry" published by Pergamon Press Co. in 1987, H. Yersin, "Photochemistry and Photophysics of Coordination Compounds" published by Springer-Verlag Co. in 1987, Akio Yamamoto, "Organometallic Chemistry -- Basis and Application" published by Shokabo Publishing Co., Ltd. in 1982, and others.
Specific examples of the ligand are preferably halogen ligands (preferably a chlorine ligand), aromatic carbon-cyclic ligands (such as a cyclopentadienyl anion, a benzene anion, or a naphthyl anion), nitrogen-containing heterocyclic ligands (such as phenylpyridine, benzoquinoline, quinolinol, bipyridyl, or phenanthroline), diketone ligands (such as acetylacetone), carboxylic acid ligands (such as an acetic acid ligand), alcoholate ligands (such as a phenolate ligand), a carbon monoxide ligand, an isonitrile ligand and a cyano ligand, and are more preferably nitrogen-containing heterocyclic ligands.
The metal complex may have, in the compound thereof, a transition metal atom, or may be what is called a dinuclear complex, which has two or more transition metal atoms. The metal complex may contain different metal atoms at the same time.

Specific examples of the emitting material include phosphorescence emitting compounds described in USP Nos. 6303238B1, and 6097147, WO00/57676, WO00/70655, WO01/08230, WO01/39234A2, WO01/41512A1, WO02/02714A2, WO02/15645A1, WO02/44189A1, JP-ANos. 2001-247859, 2002-302671, 2002-117978, 2002-225352, 2002-235076, 2003-133074 and 2002-170684, EP1211257, and JP-ANos. 2002-226495, 2002-234894, 2001-247859, 2001-298470, 2002-173674, 2002-203678, 2002-203679, 2004-357791 and 2006-256999.

(b) Fluorescence Emitting Material
General examples of the fluorescent dopant include benzoxazole, benzimidazole, benzothiazole, styrylbenzene, polyphenyl, diphenylbutadiene, tetraphenylbutadiene, naphthalimide, coumaline, pyrane, perynone, oxadiazole, aldazine, pyralidine, cyclopentadiene, bisstyrylanthracene, quinacridon, pyroropyridine, thiadiazolopyridine, cyclopentadiene, styrylamine, aromatic dimethylidyne compounds, condensed polynuclear aromatic compounds (such as anthracene, phenanthroline, pyrene, perylene, rubrene, and pentacene), various metal complexes, typical examples thereof including metal complexes of 8-quinolinol, pyrromethene complexes, and rare earth complexes, polymer compounds such as polythiophene, polyphenylene and polyphenylene vinylene, and organic silanes; and derivatives thereof.

2-2. Hole Transporting Host Material
As the hole transporting host material used in the luminescent layer in the invention, the ionization potential Ip is preferably from 5.1 to 6.4 eV, more preferably from 5.4 to 6.2 eV, even more preferably from 5.6 to 6.0 eV from the viewpoint of an improvement in endurance, and a reduction in driving voltage. The electron affinity Ea is preferably from 1.2 to 3.1 eV, more preferably from 1.4 to 3.0 eV, even more preferably from 1.8 to 2.8 eV from the viewpoint of an improvement in endurance, and a reduction in driving voltage.
The lowest triplet excitation level thereof (hereinafter abbreviated to T1) is preferably from 2.2 to 3.7 eV, more preferably from 2.4 to 3.7 eV, most preferably from 2.4 to 3.4 eV

Specific examples of the hole transporting host material include the following:
pyrrole, indole, carbazole, azaindole, azacarbazole, pyrazole, imidazole, polyarylalkanes, pyrazoline, pyrazolone, phenylenediamine, arylamines, amino-substituted chalcone, styrylanthracene, fluorenone, hydrazone, stylbene, silazane, aromatic tertiary amine compounds, styrylamine compounds, aromatic dimethylidyne compounds, porphyrin compounds, polysilane compounds, electroconductive polymer oligomers such as poly(N-vinylcarbazole), aniline copolymers, thiophene oligomers and polythiophene, organic silanes, and a carbon membrane; and derivatives thereof.
Preferred are indole derivatives, carbazole derivatives, azaindole derivatives, azacarbazole derivatives, aromatic tertiary amine compounds, and thiophene derivatives, and more preferred are materials each having in the molecule thereof plural indole skeletons, carbazole skeletons, azaindole skeletons, azacarbazole skeletons, or aromatic tertiary amine skeletons.
The hole transporting host material in the invention is in particular preferably a carbazole derivative.
According to the invention, use may be made of a host material in which hydrogen atoms in any one of the host materials are partially or wholly substituted with deuterium (see Japanese Patent Application No. 2008-126130, Japanese National Phase Publication (JP-A) No. 2004-515506).
Specific examples of compounds of the hole transporting host material include the following compounds. However, the hole transporting host material is not limited thereto.

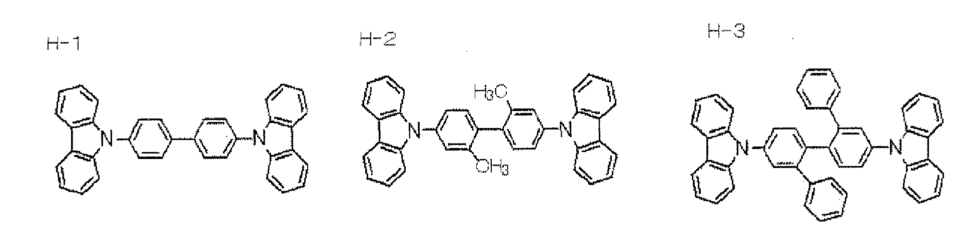

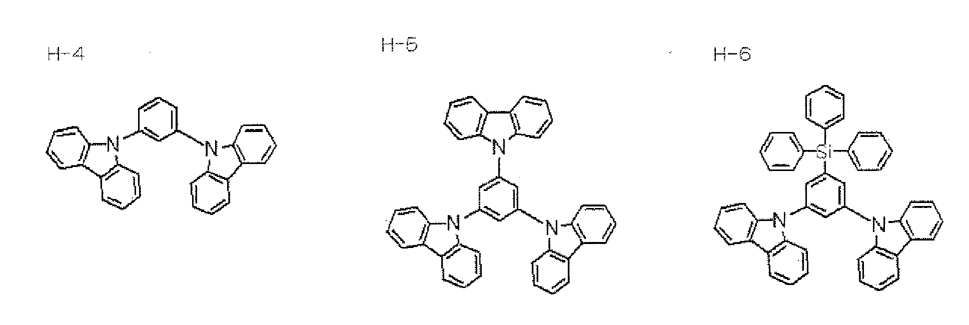

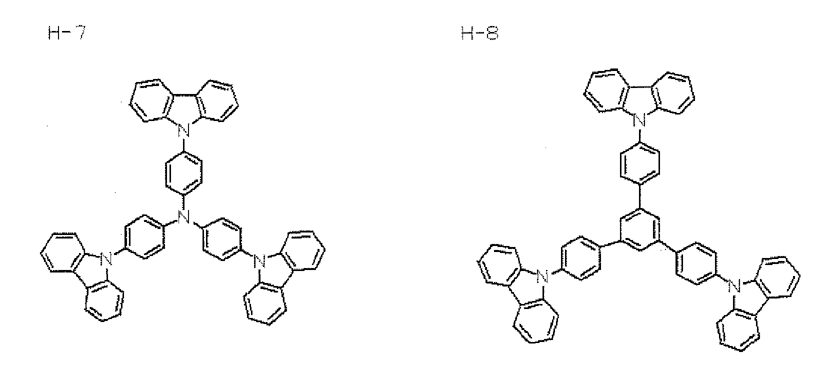

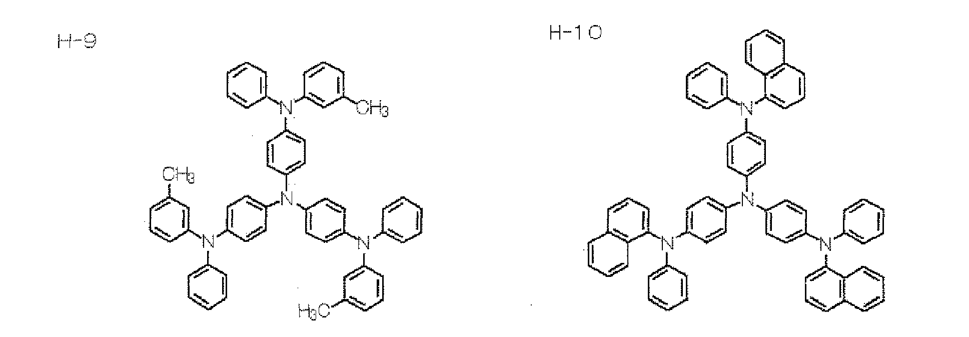

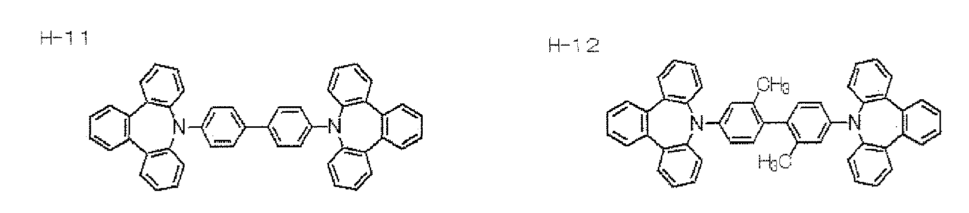

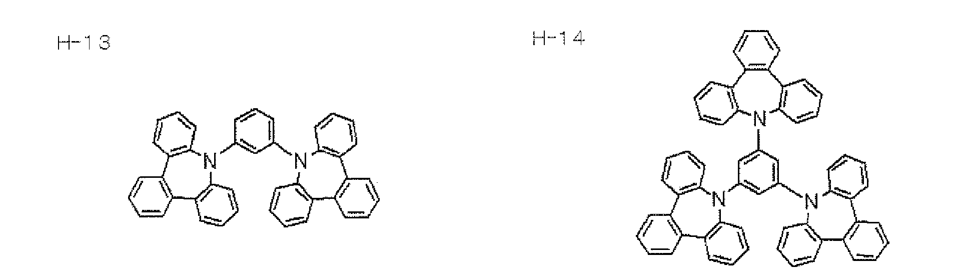

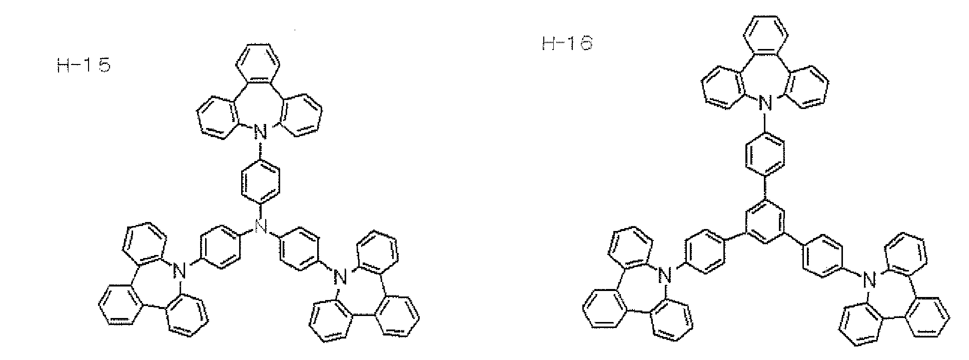

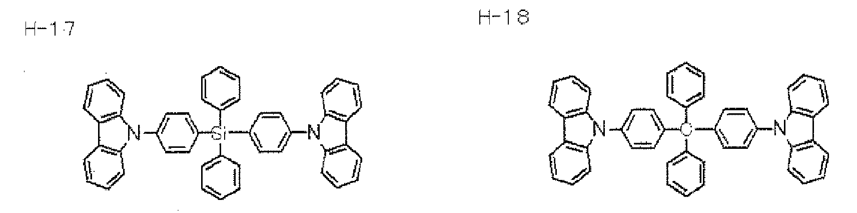

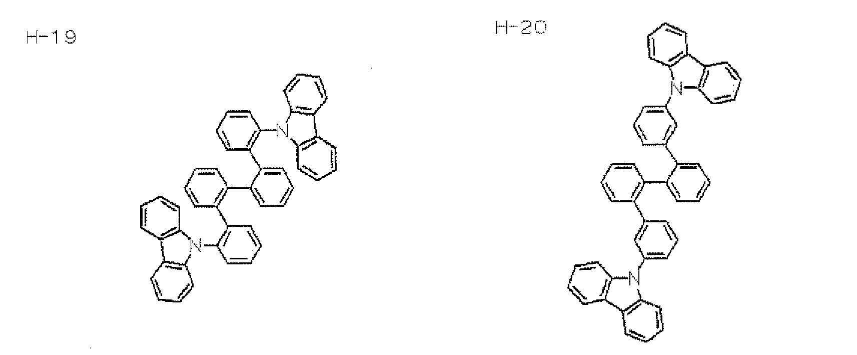

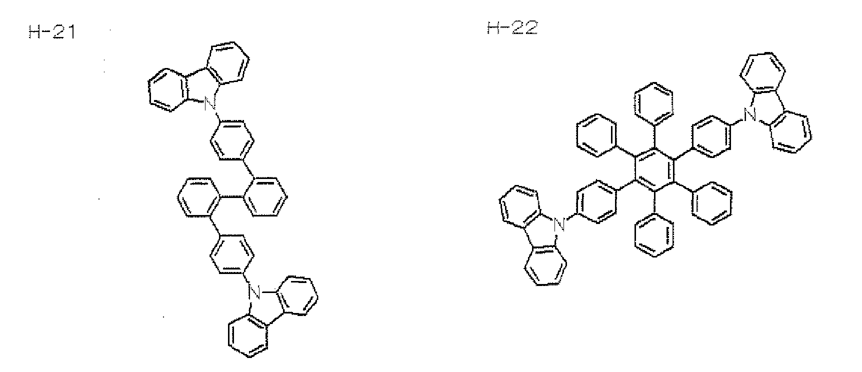

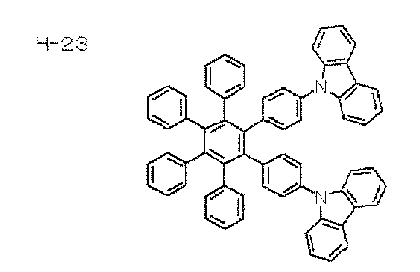

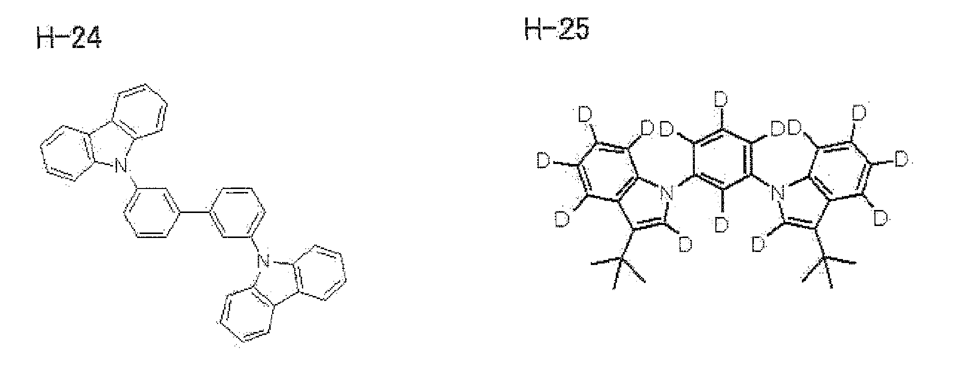

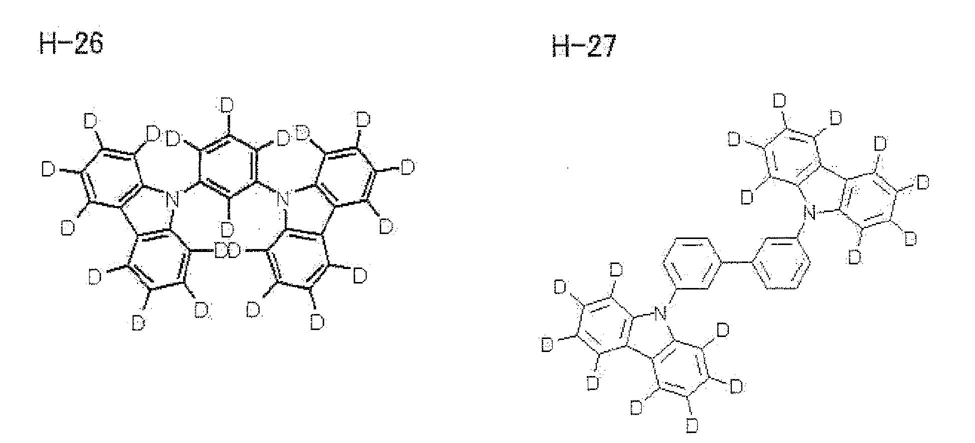

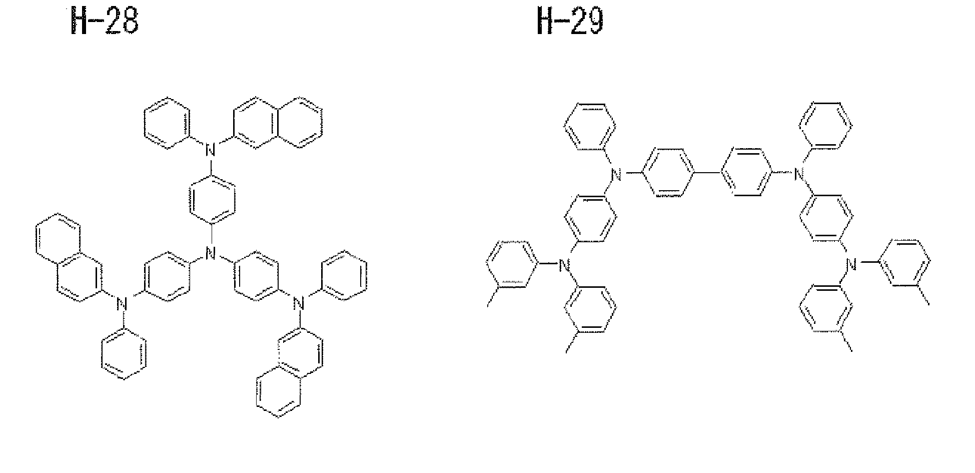

2-3. Electron Transporting Host Material
As the electron transporting host material that may be used in the invention, the electron affinity Ea thereof is preferably from 2.5 to 3.5 eV, more preferably from 2.6 to 3.4 eV, even more preferably from 2.8 to 3.3 eV from the viewpoint of an improvement in endurance, and a reduction in driving voltage. The ionization potential Ip is preferably from 5.7 to 7.5 eV, more preferably from 5.8 to 7.0 eV even more preferably from 5.9 to 6.5 eV from the viewpoint of an improvement in endurance, and a reduction in driving voltage.
The lowest triplet excitation level (hereinafter abbreviated to T1) thereof is preferably from 2.2 to 3.7 eV, more preferably from 2.4 to 3.7 eV, most preferably from 2.4 to 3.4 eV.

Specific examples of such an electron transporting host include pyridine, pyrimidine, triazine, imidazole, pyrazole, triazole, oxazole, oxadiazole, fluorenone, anthraquinodimethane, anthrone, diphenylquinone, thiopyranedioxide, carbodiimide, fluorenylidenemethane, distyrylpyrazine, fluorine-substituted aromatic compounds, tetracarboxylic anhydrides of aromatic ring such as naphthalene and perylene, and phthalocyanine; derivatives thereof (which may each be combined with a different ring to form a condensed ring); and various metal complexes, typical examples thereof including metal complexes of 8-quinolinoi derivatives, metal phthalocyanines, and metal complexes each having, as a ligand, benzoxazole or benzothiazole.

The electron transporting host is preferably a metal complex, an azole derivative (such as a benzimidazole derivative, or an imidazopyridine derivative), or an azine derivative (such as a pyridine derivative, a pyrimidine derivative, or a triazine derivative). Of these compounds, a metal complex compound is preferred in the invention from the viewpoint of endurance. The metal complex compound is more preferably a metal complex having a ligand including at least one nitrogen atom, oxygen atom or sulfur atom coordinating to a metal.
The metal ion in the metal complex is not particularly limited, and is preferably a beryllium, magnesium, aluminum, gallium, zinc, indium, tin, platinum, or palladium ion, more preferably a beryllium, aluminum, gallium, zinc, platinum, or palladium ion, even more preferably an aluminum, zinc, platinum, or palladium ion.

The ligand contained in the above-mentioned metal complex may be selected from various known ligands, and examples thereof include ligands described in H. Yersin, "Photochemistry and Photophysics of Coordination Compounds" published by Springer-Verlag Co. in 1987, Akio Yamamoto, "Organometallic Chemistry -Basis and Application-"' published by Shokabo Publishing Co., Ltd. in 1982, and others.

The ligand is preferably a nitrogen-containing heterocyclic ligand (having preferably 1 to 30 carbon atoms, more preferably 2 to 20 carbon atoms, in particular preferably 3 to 15 carbon atoms), and may be a monodentate ligand or a bi- or higher dentate ligand. The ligand is preferably any one of ligands from a bidentate ligand to a hexadentate ligand, and is also preferably a mixed ligand in which any one of ligands from a bidentate ligand to a hexadentate ligand is mixed with a monodentate ligand.
Examples of the ligand include azine ligands (such as pyridine, bipyridyl, and terpyridine ligands), hydroxyphenylazole ligands (such as hydroxyphenylbenzimidazole, hydroxyphenylbenzoxazole, hydroxyphenylimidazole, and hydroxyphenylimidazopyridine ligands), alkoxy ligands (having preferably 1 to 30 carbon atoms, more preferably 1 to 20 carbon atoms, in particular preferably 1 to 10 carbon atoms, such as methoxy, ethoxy, butoxy, and 2-ethylhexyloxy), aryloxy ligands (having preferably 6 to 30 carbon atoms, more preferably 6 to 20 carbon atoms, in particular preferably 6 to 12 carbon atoms, such as phenyloxy, 1-naphthyloxy, 2-naphthyloxy, 2,4,6-trimethylphenyloxy, and 4-biphenyloxy).

Other examples include hetero aryloxy ligands (having preferably 1 to 30 carbon atoms, more preferably 1 to 20 carbon atoms, in particular preferably from 1 to 12 carbon atoms, such as pyridyloxy, pyrazyloxy, pyrimidyloxy, and quinolyloxy), alkylthio ligands (having preferably from 1 to 30 carbon atoms, more preferably from 1 to 20 carbon atoms, in particular preferably from 1 to 12 carbon atoms, such as methylthio, and ethylthio), arylthio ligands (having preferably from 6 to 30 carbon atoms, more preferably from 6 to 20 carbon atoms, in particular preferably from 6 to 12 carbon atoms, such as phenylthio), hetero aryl thio ligands (having preferably from 1 to 30 carbon atoms, more preferably from 1 to 20 carbon atoms, in particular preferably from 1 to 12 carbon atoms, such as pyridylthio, 2-benzimidazolylthio, 2-benzoxazolylthio, and 2-benzthiazolylthin), siloxy ligands (having preferably from 1 to 30 carbon atoms, more preferably from 3 to 25 carbon atoms, in particular preferably from 6 to 20 carbon atoms, such as triphenylsiloxy, triethoxysiloxy, and triisopropylsiloxy groups), aromatic hydrocarbon anion ligands (having preferably from 6 to 30 carbon atoms, more preferably from 6 to 25 carbon atoms, in particular preferably from 6 to 20 carbon atoms, such as phenyl, naphthyl, and anthranyl anions), aromatic heterocyclic anion ligands (having preferably from 1 to 30 carbon atoms, more preferably from 2 to 25 carbon atoms, in particular preferably from 2 to 20 carbon atoms, such as pyrrole, pyrazole, pyrazole, triazole, oxazole, benzoxazole, thiazole, benzothiazole, thiophene, and benzothiophene anions), and an indolenine anion ligand. The ligand is preferably a nitrogen-containing heterocyclic ligand, an aryloxy ligand, a hetero aryloxy group, or a siloxy ligand, more preferably a nitrogen-containing heterocyclic ligand, an aryloxy ligand, a siloxy ligand, an aromatic hydrocarbon anion ligand, or an aromatic heterocyclic anion ligand.

Examples of the metal complex electron transporting host material include compounds described in JP-A Nos. 2002-235076, 2004-214179, 2004-221062, 2004-221065, 2004-221068, and 2004-327313.

Specific examples of the electron transporting host material include the following materials. However, the electron transoprting host material is not limited thereto.

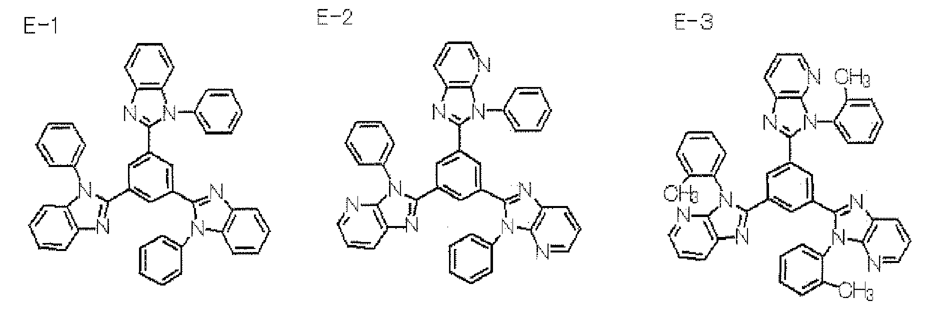

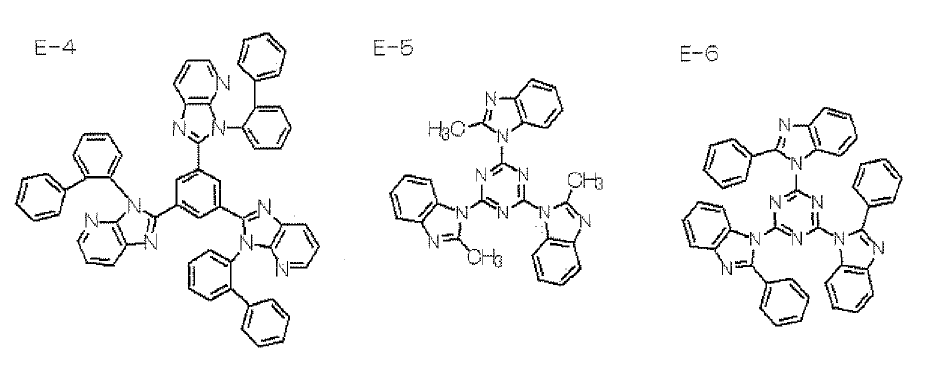

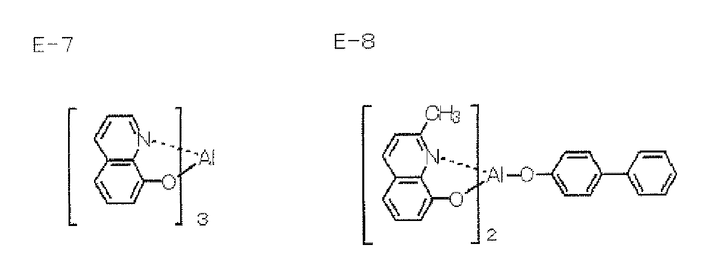

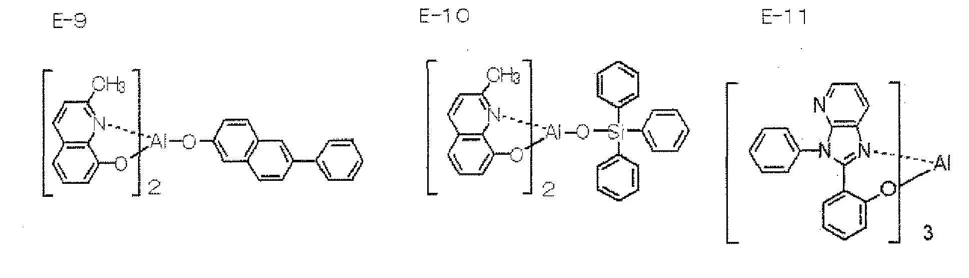

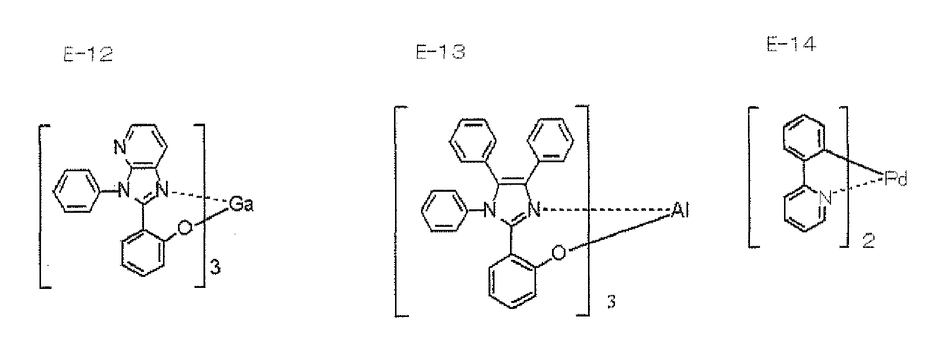

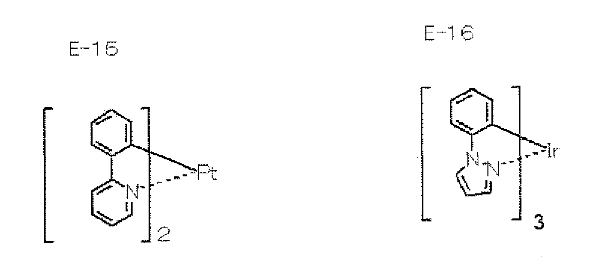

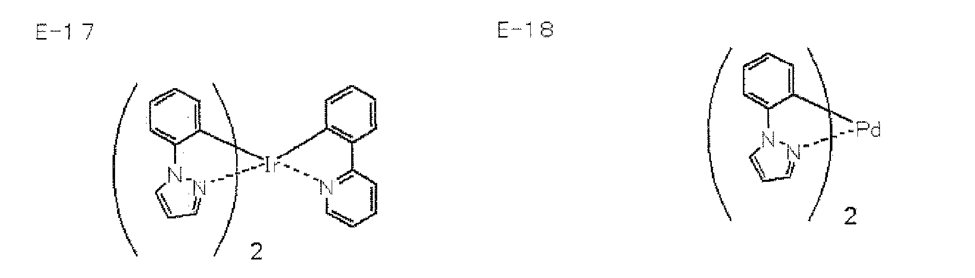

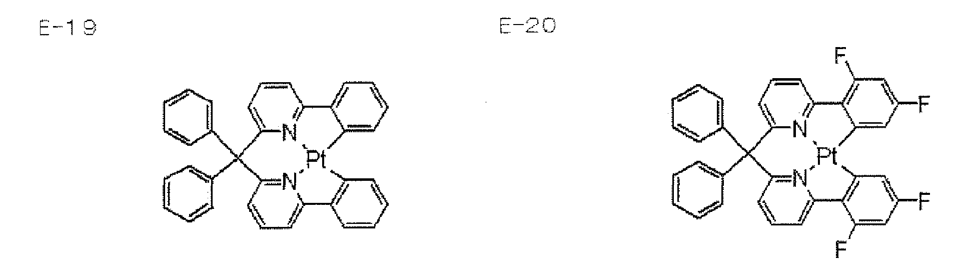

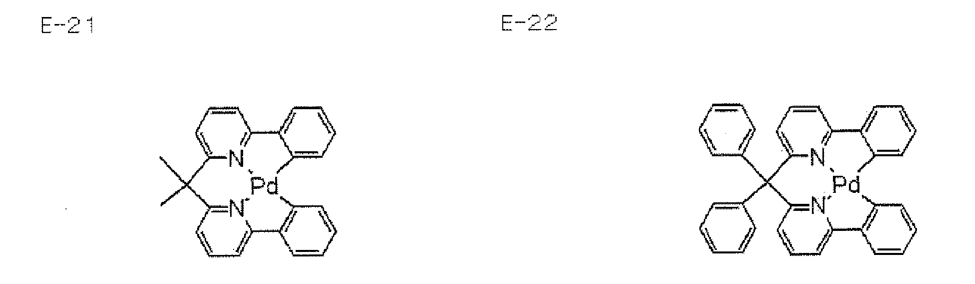

In order to make further improvements in luminescence efficiency and in endurance in the invention, two or more hole transporting hosts or electron transporting hosts may be incorporated by a method disclosed in JP-A No. 2005-233037, 2006-165526 or 2008-198801, or Japanese Patent Application No. 2007-32587. Moreover, in order to make further improvements in luminescence efficiency and in endurance in the invention, a hydrocarbon compound such as an adamantane compound may be effectively added by a method disclosed in JP-A No. 2005-294250.

In the luminescent layer in the invention, the ratio by mass of the luminescent material to the host material(s) is preferably from 50/50 to 0.1/99.9, preferably from 40/60 to 1/99, even more preferably from 30/70 to 3/97. In order to improve the luminescence efficiency and the endurance of the luminescent layer in the invention, the concentration of the luminescent material may be changed therein in sequence or step by step by a method disclosed in Japanese Patent Application No. 2007-196525, 2007-196527, 2007-196674, 2007-196675 or 2007-196676.

In the invention, two or more luminescent layers may be present. A compound represented by formula (1) or (2) may be contained in one of the layers, in two or more thereof, or in all the layers.

3. Electron Transporting Layer The electron transporting layer is a layer having a function of accepting electrons from the cathode, or the cathode side (of the EL device), and then transporting the electrons to the anode side thereof. The material usable in each of the electron injecting layer, and the electron Transporting layer in the invention is not particularly limited, and may be a low molecular compound, or a high molecular compound.
The electron transporting layer in the invention contains at least a first electron transporting layer provided so as to contact a cathode-side surface of the luminescent layer, and a second electron transporting layer provided so as to contact a cathode-side surface of the first electron transporting layer. The second electron transporting layer contains at least one electron transporting material represented by following formula (ET), and at least one selected from the group consisting of alkali metals, alkali metal salts, alkaline earth metals, and alkaline earth metal salts.

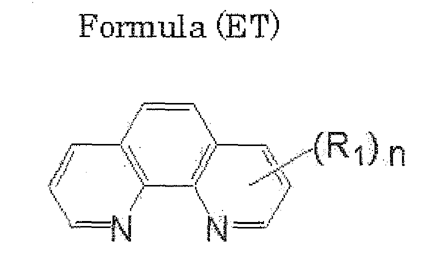

In formula (ET), R₁ each represents a hydrogen atom, or a substituent; and n represents an integer of 0 to 8 provided that when n is an integer of 2 or more, the R₁'s may be the same as or different from each other.

3-1. First Electron Transporting Layer
The materials usable in the first electron transporting layer in the invention is not particularly limited, and may be a low molecular compound or a high molecular compound.
Specifically, the layer is preferably a layer containing a pyridine derivative, a quinoline derivative, a pyrimidine derivative, a pyrazine derivative, a phthalazine derivative, a phenanthroline derivative, a triazine derivative, a triazole derivative, an oxazole derivative, an oxadiazole derivative, an imidazole derivative, a fluorenone derivative, an anthraquinodimethane derivative, an anthrone derivative, a diphenylquinone derivative, a thiopyranedioxide derivative, a carbodiimide derivative, a fluorenylidenemethane derivative, a distyrylpyrazine derivative, a tetracarboxylic anhydride of an aromatic ring such as naphthalene or perylene, a phthalocyanine derivative, any one of various metal complexes, typical examples thereof including metal complexes of 8-quinolinol derivative, metal phthalocyanines, and metal complexes each having, as a ligand, benzoxazole or benzothiazole, an organic silane derivative, a typical example thereof including silole, or some other.

The first electron transporting layer in the invention may or may not contain an electron donating dopant. Preferably, the first electron transporting layer in the invention contains no electron donating dopant.
When the first electron transporting layer contains an electron donating dopant, the electron donating dopant introduced into this layer is sufficient to have an electron donating property, thereby having a nature of reducing an organic compound. The dopant is preferably an alkali metal such as Li, an alkaline earth metal such as Mg, a transition metal containing a rare-earth metal, a reducing organic compound, or some other. The metal is in particular preferably a metal having a work function of 4.2 eV or less, and specific examples thereof include Li, Na, K, Be, Mg, Ca, Sr, Ba, Y, Cs, La, Sm, Gd, and Yb. Examples of the reducing organic compound include nitrogen-containing compounds, sulfur-containing compound, and phosphorus-containing compounds.
Besides, use may be made of materials described in JP-A Nos. 6-212153, 2000-196140, 2003-68468, 2003-229278 and 2004-342614, and others.

These electron donating dopants may be used alone or in combination of two or more. The use amount of the electron donating dopant(s), which is varied in accordance with the material(s) thereof, is preferably from 0.1 to 30% by mass, more preferably from 0.1 to 20% by mass, in particular preferably from 0.1. to 10% by mass of the electron transporting material.

The thickness of the first electron transporting layer is preferably from 1 to 500 nm, more preferably from 5 to 200 nm, even more preferably from 10 to 100 nm in order to lower the driving voltage.

3-2. Second Electron Transporting Layer
The second electron transporting layer in the invention contains at least an electron transporting material represented by formula (ET), and at least one selected from the group consisting of alkali metals, alkali metal salts, alkaline earth metals, and alkaline earth metal salts.
In the second electron transporting layer, the blend ratio by mass of the total of the selected alkali metal(s), alkaline earth metal(s), or salt(s) thereof in the electron transporting material represented by formula (ET) is preferably from 0.1 to 5.0% by mass, more preferably from 0.1 to 2.0% by mass, even more preferably from 0.1 to 1.0% by mass.

The thickness of the second electron transporting layer is preferably from 1 to 100 nm, more preferably from 2 to 30 nm, even more preferably from 5 to 20 nm in order to lower the driving voltage.

The ratio of the thickness of the first electron transporting layer to that of the second electron transporting layer ([thickness of the first electron transporting layer]/[thickness of the second electron transporting layer]) is preferably from 0.1 to 3, more preferably from 0.1 to 2, even more preferably from 0.2 to 0.5 in order to prevent a rise in voltage, and to prevent a diffusion of the alkali metal(s), alkaline earth metal(s), or the salt(s) thereof.

Electron Transporting Material Represented by formula (ET)

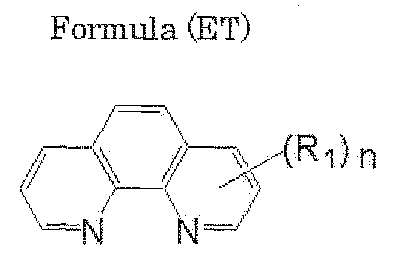

In formula (ET), R₁ each represents a hydrogen atom, or a substituent selected from the group consisting of alkyl groups having 1 to 10 carbon atoms and substituted or unsubstituted aryl groups having 6 to 30 carbon atoms; and n represents an integer of 0 to 8 provided that when n is an integer of 2 or more, the R₁'s may be the same as or different from each other.

Examples of the alkyl group having 1 to 10 carbon atoms include methyl, ethyl, n-propyl, isopropyl, n-butyl, 2-butyl, tert-butyl, n-pentyl, 2-pentyl, 3-pentyl, neopentyl, n-hexyl, 2-hexyl, 2-ethylhexyl, 2-butylhexyl, n-heptyl, n-octyl, 2-octyl, n-nonyl, and n-decyl groups.

Examples of the substituted or unsubstituted aryl group having 6 to 30 carbon atoms include phenyl, 1-naphthyl, 2-naphthyl, 4-phenyl-1-naphthyl, 1-anthryl, 2-anthryl, 9-anthryl, 10-phenyl-9-anthryl, 1-phenanthryl, 2-phenanthryl, 3-phenanthryl, 4-phenanthryl, 9-phenanthryl, 1-pyrenyl, 2-pyrenyl, 2-perylenyl, 3-perylenyl, 1-fluoranthenyl, 2-fluaranthenyl, 3-fluoranthenyl, 8-fluoranthenyl, 2-triphenytenyl 9,9-dimethylfluorene-2-yl, 9,9-dibutylfluorene-2-yl, 9,9-dihexylfluorene-2-yl, 9,9-dioctylfluorene-2-yl, 9,9-diphenylfluorene-2-yl, 2-biphenylyl, 3-biphenylyl, 4-biphenylyl, p-terphenyl-3-yl, p-terphenyl-4-yl, m-terphenyl-3-yl, m-terphenyl-4-yl, o-terphenyl-3-yl, o-terphenyl-4-yl, 4-(1-naphthyl)-1-naphthyl, o-tolyl, m-tolyl, p-tolyl, 4-tert-butylphenyl, 4-methyl-1-naphthyl, 4-phenyl-1-naphthyl, 10-methyl-9-anthryl, and 4-phenyl-8-fluoranthenyl groups.

When R₁ each represents a substituted or unsubstituted aryl group having 6 to 30 carbon atoms, the substituent thereof is preferably an alkyl group, preferably an alkyl group having 1 to 10 carbon atoms. Specific examples thereof include methyl, ethyl, n-propyl, isopropyl, n-butyl, 2-butyl, tert-butyl, n-pentyl, 2-pentyl, 3-pentyl, neopentyl, n-hexyl, 2-hexyl, 2-ethylhexyl, 2-butylhexyl, n-heptyl, n-octyl, 2-octyl, n-nonyl, and n-decyl groups.

Specific examples of the electron transporting material represented by formula (ET) include the following compounds. However, the electron transporting material is not limited thereto.

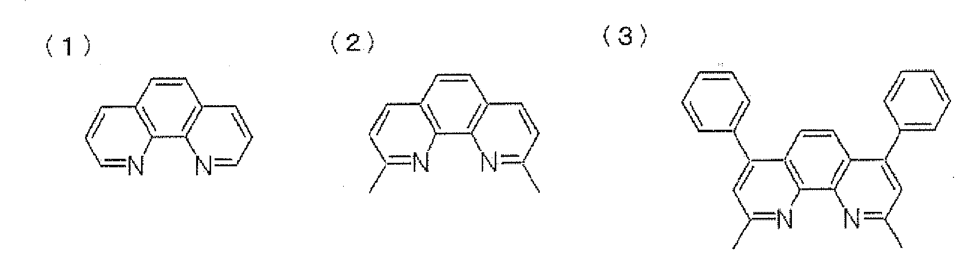

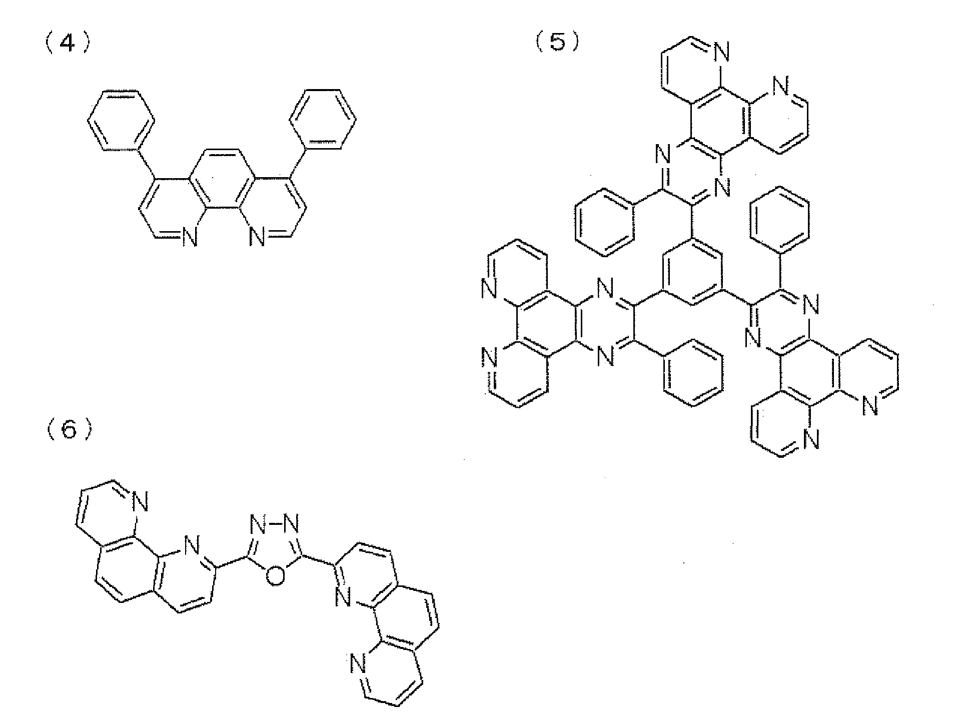

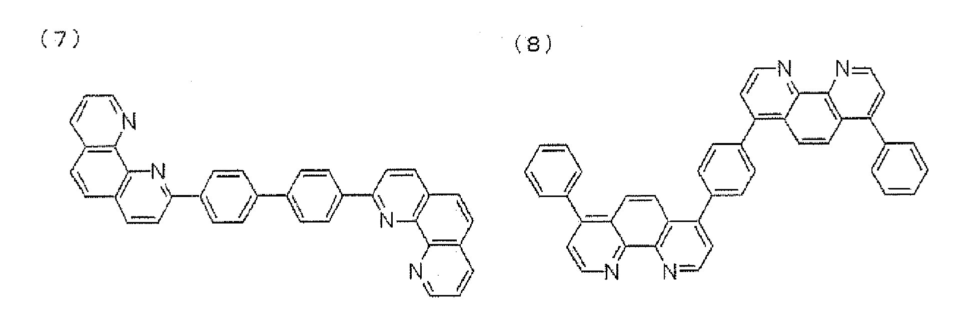

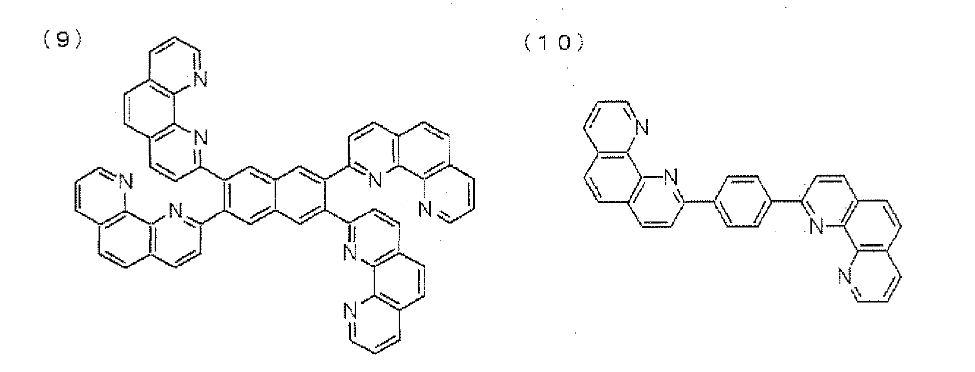

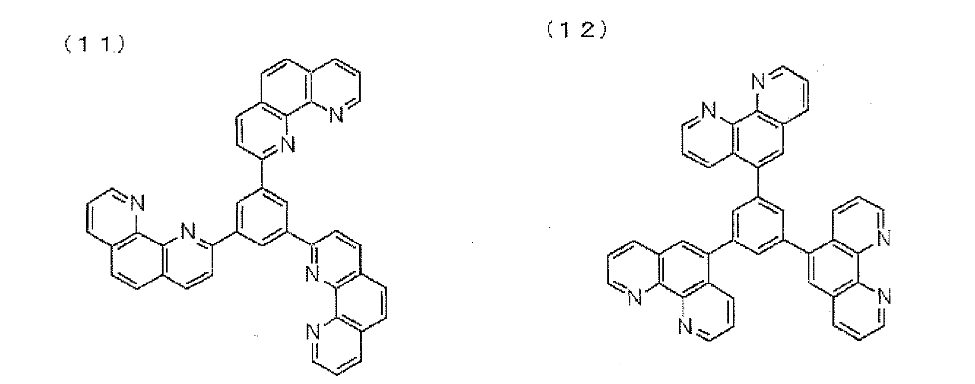

Group Consisting of Alkali Metals, Alkali Metal Salts, Alkaline Earth Metals, and Alkaline Earth Metal Salts
Examples of the alkali metals, alkali metal salts, alkaline earth metals, and alkaline earth metal salts usable in the invention include Li, Cs, Ca, LiF, NaF, KF, RbF, CsF, MgF₂, CaF₂, SrF₂, BaF₂, LiCl, NaCl, KCl, RbCl, CsCl, MgCl₂, CaCl₂, SrCl₂, BaCl₂, LiHCO₃, NaHCO₃, KHCO₃, RbHCO₃, CsHCO₃, Li₂CO₃, Na₂CO₃, K₂CO₃, Rb₂CO₃, Cs₂CO₃, MgCO₃, CaCO₃, SrCO₃, and BaCO₃. Of these metals and salts, Li or Cs is preferred.

4. Hole Injecting Layer and Hole Transporting Layer
The hole injecting layer and the hole transporting layer are each a layer having a function of accepting holes from the anode or the anode side (of the EL device) and then transporting the holes to the cathode side. The material usable in the hole injecting layer and the hole Transporting layer in the invention is not particularly limited, and may be a low molecular compound or high molecular compound, or an inorganic material.
Specifically, the layers are each preferably a layer containing a pyrrole derivative, a carbazole derivative, an imidazole derivative, a polyarylalkane derivative, a pyrazoline derivative, a pyrazolone derivative, a phenylenediamine derivative, an arylamine derivative, an amino-substituted chalcone derivative, a styrylanthracene derivative, a fluoreoone derivative, a hydrazone derivative, a stylbene derivative, a silazane derivative, an aromatic tertiary amine compound, a styrylamine compound, an aromatic dimethylidyne compound, a phthalocyanine compound, a porphyrin compound, a thiophene derivative, an organic silane derivative, carbon, a metal complex having, as a ligand, phenylazole or phenylazine, or some other. Specific examples of such a compound of the hole injecting or transporting material include compounds of H-1 to H-25. However, the compound is not limited thereto.
Examples of the inorganic material include silicon oxide, silicon dioxide, germanium oxide, germanium dioxide, germanium oxysilicide, vanadium pentaoxide, molybdenum trioxide, aluminum oxide, iron dioxide, and iron trioxide.

An electron accepting dopant may be incorporated into the hole injecting layer or the hole transporting layer in the organic EL device of the invention. In the device of the invention, it is particularly preferred to incorporate an electron accepting dopant into the hole injecting layer. The electron accepting dopant to be introduced into the hole injecting layer or the hole transporting layer may be an inorganic compound or organic compound as far as the compound has an electron accepting performance, thereby having a nature of oxidizing an organic compound.

Specific examples of the inorganic compound include metal halides such as ferric chloride, aluminum chloride, gallium chloride, indium chloride, and antimony pentachloride; and metal oxides such as vanadium pentaoxide, and molybdenum trioxide.

When the electron accepting dopant is an organic compound, use is preferably made of a compound having a nitro group, a halogen, a cyano group, a trifluoromethyl group or some other group as a substituent, a quinone-based compound, an acid anhydride compound, a fullerene, or some other. Specific examples of the organic electron donor include hexacyanobutadiene, hexacyanobenzene, tetracyanoethylene, tetracyanoquinodimethane, tetrafluorotetracyanoquinodimethane, p-fluoranil, p-chloranil, p-bromanil, p-benzoquinone, 2,6-dichlorobenzoquinone, 2,5-dichlorobenzoquinone, tetramethylbenzoquinone, 1,2,4,5-tetracyanobenzene, o-dicyanobenzene, p-dicyanobenzene, 1,4-dicyanotetrafluorobenzene, 2,3-dichloro-5,6-dicyanobenzoquinone, p-dinitrobenzene, m-dinitrobenzene, o-dinitrobenzene, p-cyanonitrobenzene, m-cyanonitrobenzene, o-cyanonitrobenzene, 1,4-naphthoquinone, 2,3-dichloronaphthoquinone, 1-nitronaphthalene, 2-nitronaphthalene, 1,3-dinitronaphthalene, 1,5-dinitronaphthalene, 9-cyanoanthracene, 9-nitroanthracene, 9, 10-anthraquinone, 1,3,6,8-tetranitrocarbazole, 2,4,7-trinitro-9-fluorenone, 2,3,5,6-tetracyanopyridine, maleic anhydride, phthalic anhydride, C60, and C70.

Of these examples, preferred are hexacyanobutadiene, hexacyanobenzene, tetracyanoethylene, tetracyanoquinodimethane, tetrafluorotetracyanoquinodimethane, p-fluoranil, p-chloranil, p-bromanil, p-benzoquinone, 2,6-dichlorobenzoquinone, 2,5-dichlorobenzoquinone, 1,2,4,5-tetracyanobenzene, 1,4-dicyanotetrafluorobenzene, 2,3-dichloro-5,6-dicyanobenzoquinone, p-dinitrobenzene, p-dinitrobenzene, o-dinitrobenzene, 1,4-naphthoquinone, 2,3-dichloronaphthoquinone, 1,3-dinitronaphthalene, 1,5-dinitronaphthalene, 9,10-anthraquinone, 1,3,6,8-tetranitrocarbazole, 2,4,7-trinitro-9-fluorenone, 2,3,5,6-tetracyanopyridine, or C60, and particularly preferred are hexacyanobutadiene, hexacyanobenzene, tetracyanoethylene, tetracyanoquinodimethane, tetrafluorotetracyanoquinodimethane, p-fluoranil, p-chloranil, p-bromanil, 2.6-dichlorobenzoquinone, 2,5-dichlorobenzoquinone, 2,3-dichloranaphthoquinone, 1,2,4,5-tetracyanobenzene, 2,3-diehloro-5,6-dicyanabenzoquinone, or 2,3,5,6-tetracyanopyridine.

These organic electron donors may be used alone or in combination of two or more thereof.
Besides, compounds described in the following may be preferably used: JP-A Nos. 6-212153,11-111463, 11-251067, 2000-196140, 2000-286054, 2000-315580, 2001-102175, 2001-160493, 2002-252085, 2002-56985, 2003-157981, 2003-217862, 2003-229278, 2004-342614, 2005-72012, 2005-166637, and 2005-209643.

These electron accepting dopants may be used alone or in combination of two or more thereof. The use amount of the electron accepting dopant(s), which is varied in accordance with the material(s) thereof, is preferably from 4.01 to 50% by mass, more preferably from 0.05 to 20% by mass, in particular preferably from 0.1 to 10% by mass of the hole transporting material.

The thickness of the hole injecting layer and that of the hole transporting layer are each preferably 500 nm or less in order to lower the driving voltage.
The thickness of the hole transporting layer is preferably from 1 to 500 nm, more preferably from 5 to 300 nm, even more preferably from 10 to 200 nm. The thickness of the hole injecting layer is preferably from 0.1 to 500 nm, more preferably from 0.5 to 300 nm, even more preferably from 1 to 200 nm.
The hole injecting layer and the hole transporting layer may each preferably have a mono-layered structure made of one or more of the above-mentioned materials, or a multi-layered structure composed of plural layers having the same composition or having different compositions.
In order to make an improvement in luminescence efficiency in the invention, an electrically inactive hydrocarbon compound such as an adamantane compound may be added to the hole transporting layer by a method disclosed in JP-A No. 2005-294249 and others.
The T1 of the hole injecting layer and that of the hole transporting layer are not particularly limited. In order to restrain the diffusion of excitons, the difference in T1 between the luminescent layer and the hole transporting layer adjacent to the luminescent layer is preferably 1 eV or less.

3. Structure of the Organic EL Device
The following describes the structure of the organic EL device of the invention in detail.
The organic compound layer in the invention has, from the anode side (of the device), the luminescent layer, the first electron transporting layer, and the second electron transporting layer, and is preferably an embodiment wherein the hole transporting layer, the luminescent layer, the first electron transporting layer, and the second electron transporting layer are laminated in this order therefrom. The device may have charge blocking layers such as an electron blocking layer, a hole blocking layer) and/or some other layer between the hole transporting layer and the luminescent layer or between the luminescent layer and the electron transporting layers.

The EL device may have a hole injecting layer between the anode and the hole transporting layer, and may have an electron injecting layer between the cathode and the electron transporting layers. Each of the layers may be divided into plural secondary layers.

In order to inject charges more effectively to the luminescent layer in the invention, a stepped layer may be effectively provided by a method disclosed in JP-A No. 2006-279014 or 2006-351680. Specifically, in the stepped layer, its hole transporting layer is composed of plural layers including a layer adjacent to the luminescent layer, and a relationship of Ip1 > Ip2 > Ip3 is satisfied wherein Ip1 represents the ionization potential of the luminescent layer. Ip2 represents the ionization potential of the hole transporting layer adjacent to the luminescent layer, and Ip3 represents the ionization potential of the other hole transporting layer while its electron transporting layer is composed of plural layers including a layer adjacent to the luminescent layer, and a relationship of Ea1 < Ea2 < Ea3 is satisfied wherein Ea1 represents the electron affinity of the luminescent layer, Ea2 represents the electron affinity of the electron transporting layer adjacent to the luminescent layer, and Ea3 represents the electron affinity of the electron transporting layer.
The stepped layer may be made thin by a method disclosed in JP-A No. 2006-351715, thereby injecting charges more effectively into the concerned layer.

In order to inject electrons from the cathode more effectively to the (primary) electron transporting layer to lower the voltage, a thin organic layer may be provided between the cathode and the electron transporting layer by a method disclosed in JP-A No. 2007-227888.

To make the luminescence efficiency better in the invention, a charge generating layer may be provided by a method disclosed in JP-A No. 2003-272860 or 11-329748, thereby producing a multi-photon type device.

To make the device of the invention better in luminescence efficiency, endurance and chromaticity, it is allowable to lay organic compound layers containing the luminescent layer between a reflecting plate (or reflecting electrode) and a translucent electrode, and make the device into a resonator structure by a method disclosed in JP-A No. 8-213174, 11-126691, 2002-367770 or 21304-127795, Japanese Patent No. 2830474, or some other.

<Substrate>
As substrates used in the invention, the substrate on the luminescent-device side where light emitted from the luminescent layer is taken out is preferably a substrate which does not cause the light to be scattered or attenuated. Specific examples of the material thereof include inorganic materials, such as zirconia-stabilized yttrium (YSZ), and glass; organic materials, such as polyesters such as polyethylene terephthalate, polybutylene phthalate and polyethylene naphthalate, polystyrene, polycarbonate, polyethersulfone, polyarylate, polyimide, polycycloolefin, norbornene resin, and poly(chlorotrifluoroethylene).
In the case of using, for example, a glass piece as the substrate, the material thereof is preferably non-alkali glass for a decrease in the quantity of eluted-out ions from the glass. When a soda-lime glass piece is used, it is preferred to use the glass piece coated with a barrier coat made of silica or some other. In the case of using an organic material, it is preferred that the material is excellent in heat resistance, dimensional stability, solvent resistance, electrical insulation, and workability.

The shape or form, the structure, the size and other factors of the substrate are not particularly limited, and may be appropriately selected in accordance with the usage and purpose of the luminescent device, and others. In general, the form of the substrate is preferably a plate form. The structure of the substrate may be a monolayered structure or a laminated structure. The substrate may be made of a single member, or two or more members.

In the substrate, a moisture-permeation resisting layer (gas barrier layer) may be provided onto the front or rear surface thereof.
The material of the moisture-permeation resisting layer (gas barrier layer) is preferably an organic substance such as silicon nitride or silicon oxide. The moisture-permeation resisting layer (gas barrier layer) may be formed by, for example, a high-frequency sputtering method.
When a thermoplastic substrate is used, a hard coat layer, an undercoat layer or some other layer may be further provided if necessary.
The substrate may be washed and/or pre-treated before or after the electrodes are formed, or before the formation of the organic compound layers. The washing may be conducted with any one of water, pure water, ion exchange water, an acid, an alkaline water, and organic solvents. The substrate may be immersed and washed by ultrasonic waves. The pre-treatment may be conducted in order to decompose and remove organic substances, improve the substrate in adhesiveness, or promote the injection of charges from the electrodes to the organic compound layers. The method for the pre-treatment is preferably UV ozone treatment, oxygen plasma treatment, or some other. However, the method is not particularly limited.

<Anode>
The anode is sufficient to have a function as an electrode for supplying holes to the organic compound layers. The shape, the structure, the size, and other factors thereof are not particularly limited. The material of the anode may be appropriately selected from known electrode materials in accordance with the usage and the purpose of the luminescent device. As described above, the anode is formed as a transparent electrode when the anode is on the side (of the device) where light is taken out. When the anode is formed on the side opposite to the light-taking-out side, the anode may or may not be transparent.

The material of the anode is preferably, for example, a metal, an alloy, a metal oxide or an electroconductive compound, or any mixture thereof. Specific examples of the anode material include electroconductive metal oxides, such as a tin oxide doped with antimony or fluorine (ATO or FTO), tin oxide, zinc oxide, indium oxide, indium tin oxide (ITO), and indium zinc oxide (IZO); metals such as gold, silver, chromium, and nickel; mixtures or laminates each composed of any one or more of these metals and any one or more of the electroconductive metal oxides; inorganic electroconductive substances such as copper iodide, and copper sulfide; organic electroconductive materials such as polyaniline, polythiophene, and polypyrrole; and laminates each composed of ITO and any one or more of these electroconductive materials. Of these examples, electroconductive metal oxides are preferred. ITO is particularly preferred from the viewpoint of productivity, high electroconductivity, transparency, and others. ITO may be combined with any other material to form a laminate, or an auxiliary electrode or the like may be added to the ITO electrode.

The anode may be formed on the substrate in accordance with a method selected appropriately from wet methods such as printing and coating, physical methods such as vacuum evaporation, sputtering, and ion plating, and chemical methods such as CVD, and plasma CVD, considering the adaptability thereof to the material constituting the anode. In the case of selecting, for example, ITO as the material of the anode, the anode may be formed by DC or high-frequency sputtering, vacuum evaporation, ion plating or some other.
The work function of the anode is not particularly limited as far as the work function permits holes to be injected into the hole transporting layer or hole injecting layer adjacent to the anode. The work function is preferably from 4.0 to 6.0 eV, more preferably from 4.5 to 5.8 eV
The work function of the anode may be adjusted into any value by UV ozone treatment or oxygen plasma treatment.

In the organic electroluminescent device of the invention, the position where the anode is formed is not particularly limited, and may be appropriately selected in accordance with the usage and the purpose of the luminescent device. Preferably, the anode is formed on the substrate. In this case, the anode may be formed on the whole of one of both surfaces of the substrate, or on a partial area thereof.

The patterning for forming the anode may be conducted by chemical etching by use of photolithography or the like, or physical etching by use of a laser, or the like. Moreover, the patterning may be conducted by vacuum evaporation or sputtering in the state that a mask is put onto a workpiece, or may be conducted by a lift-off method, or a printing method.

The thickness of the anode may be appropriately selected in accordance with the material constituting the anode, and cannot be specified without reservation. The thickness is usually from about 10 nm to 50 µm, preferably from 50 nm to 20 µm.

The resistance value of the anode is preferably 10³ Ω/□ or less, more preferably 10² Ω/□ or less. When the anode is transparent, the anode may be colorless and transparent, or colored and transparent. In order to take out luminescence from the transparent anode side, the transmissibility thereof is preferably 60% or more, more preferably 70% or more.

About transparent anodes, a detailed description is made in "New Development of Transparent Electrode Film" supervised by Yutaka Sawada and published by CMC Publishing Co., Ltd. (1999), and any matter described therein may be applied to the invention. In the case of using a plastic substrate low in heat resistance, it is preferred to use a transparent anode made by forming ITO or IZO into a film at a low temperature of 150°C or lower.

<Cathode>
The cathode is usually sufficient to have a function as an electrode for injecting electrons to the organic compound layers. The shape, the structure, the size, and other factors thereof are not particularly limited. The material of the cathode may be appropriately selected from known electrode materials in accordance with the usage and the purpose of the luminescent device. When the cathode side of the luminescent device is a light-taking-out side, the cathode is preferably transparent or translucent.

The material constituting the cathode is, for example, a metal, an alloy, a metal oxide or an electroconductive compound, or any mixture thereof. Specific examples thereof include alkali metals (such as Li, Na, K and Cs), alkaline earth metals (such as Mg and Ca), gold, silver, lead, aluminum, sodium-potassium alloy, lithium-aluminum alloy, magnesium-silver alloy, indium, and rare earth metals such as ytterbium. These may be used alone. In order to make the cathode compatible between stability and electron-injecting performance, two or more thereof may be appropriately used together in the state of being co-evaporated or laminated.
The work function of the cathode is not particularly limited as far as the work function permits electrons to be injected into the organic compound layers adjacent thereto. The work function is preferably from 2.5 to 4.5 eV, more preferably from 2.5 to 4.3 eV.

Of these materials, alkali metals and alkaline earth metals are each preferred as the cathode-constituting material from the viewpoint of electron injecting performance. A material made mainly of aluminum is preferred since the material is excellent in storage stability. The material may be combined with an electroconductive metal oxide such as ITO to form a laminated structure.
The material made mainly of aluminum denotes aluminum alone, or any alloy or mixture of aluminum, and 0.01 to 10% by mass of an alkali metal or alkaline earth metal (for example, lithium-aluminum alloy, or magnesium-aluminum alloy).

About materials of a cathode, a detailed description is made in JP-A Nos. 2-15595 and 5-121172. Materials described in these publications may be applied to the invention.

The method for forming the cathode is not particularly limited, and may be a known method. Examples thereof include wet methods such as printing and coating, physical methods such as vacuum evaporation, sputtering, and ion plating, and chemical methods such as CVD, and plasma CVD. Of these methods, an appropriate method is selected, considering the adaptability thereof to the cathode-constituting material. In accordance with the selected method, the cathode may be formed. In the case of selecting, for example, a metal or the like as the material of the cathode, the cathode may be formed by subjecting one or more species thereof simultaneously or sequentially to sputtering or some other.

The patterning for forming the cathode may be conducted by chemical etching by use of photolithography or the like, or physical etching by use of a laser, or the like. Moreover, the patterning may be conducted by vacuum evaporation, sputtering or some other method in the state that a mask is put onto a workpiece, or may be conducted by a lift-off method, or a printing method.

In the invention, the position where the cathode is formed is not particularly limited, and the cathode may be formed on the whole or a partial area of the top of the organic compound layers.
A dielectric layer may be interposed with a thickness of 0.1 to 5 nm between the cathode and the organic compound layers, the dielectric layer being made of a fluorite of an alkali metal or alkaline earth metal, an oxide, or some other. This dielectric layer may be regarded as a kind of electron injecting layer. The dielectric layer may be formed by, for example, vacuum evaporation, sputtering, ion plating, or some other method.

The thickness of the cathode may be appropriately selected in accordance with the cathode-constituting material, and may not be specified without reservation. The thickness is usually from about 5 nm to 5 µm, preferably from 10 nm to 1 µm.
The cathode may be transparent or opaque. The transparent cathode may be formed by forming the material of the cathode into a thin film having a thickness of 1. to 10 nm, and further laminating a transparent electroconductive material such as ITO or IZO thereon.
In the invention, the EL device may be rendered a top emission type device by making the anode side thereof opaque (a reflecting electrode) and making the cathode side transparent or translucent. The EL device may be rendered a bottom emission type device by making the anode side thereof transparent and making the cathode side opaque (a reflecting electrode). The device may be made into a both-sided light emission type by making both of the anode and the cathode transparent.

<Organic compound layers>
A description is made about the organic compound layers in the invention.
In the organic EL device of the invention, organic compound layers other than the luminescent layer and the electron transporting layers include a hole transporting layer, a charge blocking layer, a hole injecting layer, and an electron injecting layer.

-Formation of the organic compound layers-In the organic luminescent device of the invention, each of the layers constituting the organic compound layers may be preferably formed by any one of dry film-forming methods such as evaporation (i.e., vapor-deposition) and sputtering, a wet coating method, a transcribing method, a printing method, and an ink-jetting method.
Out of the dry film-forming methods, vapor deposition is mainly used. The film-forming rate in the vapor deposition of each of the organic compound layers is preferably from 0.1 to 100 angstroms/second, more preferably from 0.1 to 50 angstroms/second. The heating temperature therein is not limited as far as the temperature is in the range of temperatures that do not cause a decomposition of the material.
The vacuum degree in the film-formation by the vapor deposition is preferably from 10⁻² to 10⁻⁹ Pa, more preferably from 10⁻³ to 10⁻⁸ Pa.

The vacuum atmosphere in the vapor-deposition film-formation may be either an air vacuum atmosphere or a vacuum atmosphere of an inert gas such as nitrogen or argon.
Before or at the time of the vapor-deposition film-formation, water and oxygen inside a tank for the vapor deposition may be removed by conducting baking treatment or heating a getter agent or the like.
At the time of or during the vapor-deposition film-formation, water adhering to the substrate(s) may be removed or the film quality of the organic film(s) may be controlled by cooling or heating the substrate(s).
In the case of forming the luminescent layer by vapor deposition, a host material and a luminescent material may be put into different evaporation sources, respectively, so as to be subjected to co-evaporation, or may be incorporated into a single evaporation source so as to be evaporated and formed into a film.
In order to heat the evaporation source(s) evenly, a thermo ball or the like may be set to each of the source(s).
Alter the film is formed, the film may be thermally treated. The temperature for the thermal treatment is not particularly limited, and may be set to the glass transition point of the constituting material or higher, or lower than the point as required.

Furthermore, the individual films are formed, and sealed, and subsequently the workpiece may be thermally treated. The temperature for the thermal treatment is not particularly limited, and may be set to the glass transition temperature of the constituting materials or higher, or lower than the temperature as required.

<Protective layer>
In the invention, the whole of the organic EL device may be protected by a protective layer.
A material contained in the protective layer is sufficient to have a function of restraining water, oxygen and other materials causing the promotion of a deterioration in the device from entering the inside of the device.
Specific examples thereof include metals such as In, Sn, Pb, Au, Cu, Ag, Al. Ti and Ni, metal oxides such as MgO, SiO, SiO₂, Al₂O₃, GeO, NiO, CaO, BaO, Fe₂O₃, Y₂O₃ and TiO₂, metal nitrides such as SiNₓ and SiNₓO_{y}, metal fluorides such as MgF₂, LiF, AlF₃ and CaF₂, polyethylene, polypropylene, polymethyl methacrylate, polyimide, polyurea, polytetrafluoroethylene, polychlorotrifluoroethylene, polydichlorodifluoroethylene, a copolymer made from chlorotrifluroethylene and dichlorodifluoroethylene, a copolymer obtained by copolymerizing tetrafluoroethylene and a monomer mixture containing at least one comonomer, a fluorine-containing copolymer having, in the copolymer main chain thereof, a cyclic structure, a water absorbent having a water absorption of 1 % or more, and a moisture-proof material having a water absorption of 0.1% or less.

The method for forming the protective layer is not particularly limited. For example, the following may be used: vacuum evaporation, sputtering, reactive sputtering, an MBE (molecular beam epitaxial) method, a cluster ion beam method, an ion plating method, a plasma polymerizing method (high-frequency excited ion plating method), plasma CVD, laser CVD, thermal CVD, gas source CVD, a coating method, a printing method, or a transcribing method.

<Sealing>
In the organic electroluminescent device of the invention, the whole of the device may be sealed with a sealing container, or may be sealed with an inorganic film made of SiN, SiON or some other. The whole may be solid-sealed by a method disclosed in JP-A No. 2005-294249 and others.
A water absorbent or an inert liquid may be air-tightly put into the space between the sealing container and the luminescent device. The water absorbent is not particularly limited, and examples thereof include barium oxide, sodium oxide, potassium oxide, calcium oxide, sodium sulfate, calcium sulfate, magnesium sulfate, phosphorus pentaoxide, calcium chloride, magnesium chloride, copper chloride, cesium fluoride, niobium fluoride, calcium bromide, vanadium bromide, a molecular sieve, zeolite, and magnesium oxide. The inert liquid is not particularly limited and examples thereof include paraffins, fluid paraffins, fluorine-containing solvents such as perfluoroalkanes, perfluoroamines and perfluoroether, chlorine-containing solvents, and silicone oils.

<Driving>
In the organic electroluminescent device of the invention, luminescence may be given by applying a DC (which may optionally contain an alternating current component) voltage (of 2 to 15 V usually), or a direct current to its anode and its cathode across these electrodes.
In the method for driving the organic electroluminescent device of the invention, use may be made of driving methods described in individual specifications of JP-A Nos. 2-148687, 6-301355, 5-29080, 7-134558, 8-234685 and 8-241047, Japanese Patent No. 2784615, USP Nos. 5828429 and 6023308, and others.
The device of the invention may be thermally treated by a method disclosed in Japanese Patent Application No. 2008-48630 and others in order to drive the device more stably after the production of the device. The device may be subjected to electric current treatment by a method disclosed in JP-A No. 8-185979, and others.
In the case of driving the device actively through a TFT, the TFT may be any one of amorphous silicon, low-temperature polysilicon, and an oxide semiconductor.

The luminescent device of the invention may be improved in light-taking-out efficiency by known various methods. For example, the device may be improved in light-taking-out efficiency to be improved in external quantum efficiency by working the form of a surface or surfaces of its substrate(s) (for example, forming a pattern or patterns of fine irregularities in the surface(s)), by controlling the refractive index(es) of the substrate(s), its ITO layer and/or the organic compound layers, by controlling the film thickness(es) of the substrate(s), the ITO layer and/or the organic compound layers, or by performing some other operation.
The device may be further improved in chromaticity by setting up a color filter, or using a color converting material.
By adding, to the device of the invention, a luminescent material capable of emitting light in some other color, a different color, a typical example thereof being white, may be reproduced. In this case, it is allowable to use a monolayered luminescent layer or plural luminescent layers. The device of the invention may be rendered a multi-photon type device.
The device of the invention may be combined with one or more different pixels in a panel, whereby many colors may be reproduced. In this case, the device may be combined with secondary pixels in three colors of red, green and blue. It may be decided in accordance with the purpose of the device what color the color of the device is combined with.
The method for driving the panel may be active driving or passive driving, and may be current driving or voltage driving.

Usage of the Invention:
The organic electroluminescent device of the invention may be favorably used for a display device, a display, a backlight, electrophotography, an illumination source, a recording light source, an exposing light source, a reading-out light source, an indicator, a signboard, an interior, optical communication, and others.

### EXAMPLES

The invention is specifically described by way of the following examples; however, the invention is not limited to these examples.

Example 1
1. Production of organic EL devices
1) Device 1 of the invention
Prepared as a transparent supporting substrate was a product (manufactured by Tokyo Sanyo Vacuum Industries Co., Ltd.) yielded by evaporating and depositing indium tin oxide (hereinafter abbreviated to ITO) into the form of a film having a thickness of 100 nm on a glass substrate of 25 mm x 25 mm x 0.7 mm in size. This transparent supporting substrate was etched and washed.
On this substrate, the following organic compound layers were successively formed by vacuum evaporation at a film-forming rate of 1 angstrom per second.

Hole injecting layer: 4,4',4"-tris(2-naphthylphenylamino)triphenylamine (abbreviated to 2-TNATA), and 2,3,5 ,6-tetrafluoro-7,7,8,8-tetracyanoquinodimethane (abbreviated to F4-TCNQ) were co-evaporated and deposited to set the amount of F4-TCNQ to 1.0% by mass of 2-TNATA. The thickness was 120 nm.
Hole transporting layer: the hole transporting material used therefor was N,N'-dinaphthyl-N,N'-diphenyl-[1,1'-biphenyl]-4,4'-diamine (abbreviated to α-NPD). The thickness was 10 nm.

Electron blocking layer: the following compound "a" was evaporated and deposited into a thickness of 3 nm.

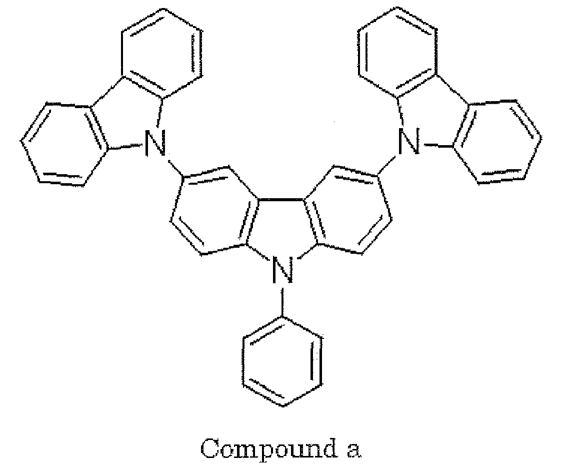

Luminescent layer: 1,3-bis(N-carbazol-9-yl)benzene (abbreviated to mCP) as a host material, and a platinum complex Pt-1 as a luminescent material were co-evaporated and deposited to give a ratio by mass of mCP to Pt-1 to 85/15. The thickness was 30 nm.

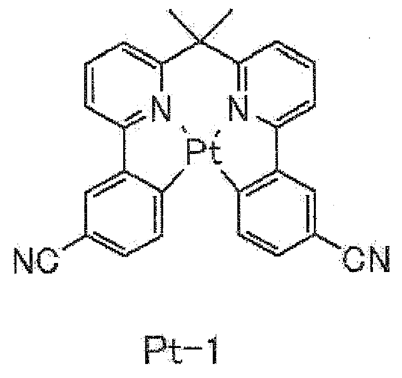

First electron transporting layer: Aluminum (III) bis(2-methyl-8-quinolato)-4-phenylphenolate (abbreviated to BAlq) was evaporated and deposited into a thickness of 10 nm.
Second electron transporting layer: Bathocuproine (abbreviated to BCP) illustrated below as a compound represented by formula (ET), and Li were co-evaporated and deposited to set the dope amount oF Li to 0.4% by mass of BCP. The thickness was 20 nm.

Furthermore, LiF was evaporated and deposited into an electron injecting layer having a thickness of 1 nm. Thereafter, while a shadow mask was used to perform patterning, Al was deposited, as a cathode, into a thickness of 100 nm by vacuum evaporation.

The produced laminate was put into a glove box purged with nitrogen gas, and then sealed with a sealing can made of glass through an ultraviolet curable adhesive (trade name: XNR5516HV, manufactured by Nagase-Chiba Ltd.).

Bathocuproine

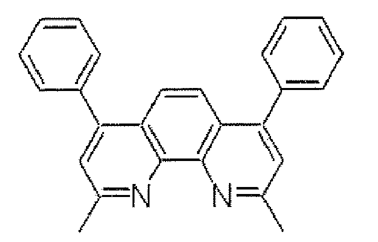

2) Device 2 of the invention
In the same way as in the formation of the device 1 of the invention, an device 2 of the invention was produced which had the same composition as the device 1 of the invention except that mCP in the luminescent layer was changed to the following hole transporting host material A.

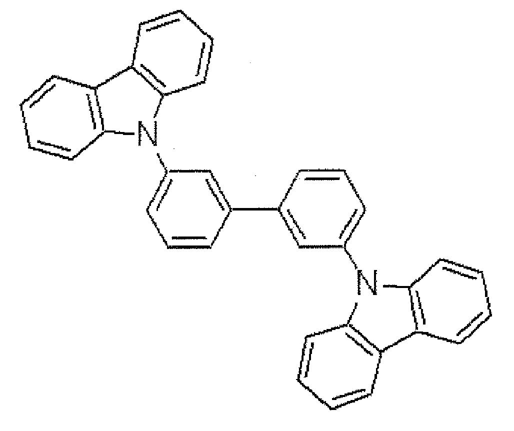

Hole transporting host materil A

3) Devices 3, 5 and 6 of the invention
In the same way as in the formation of the device 1 of the invention, devices 3, 5 and 6 of the invention were produced which each had the same composition as the device 1 of the invention except that the dope amount of Li relative to the amount of BCP in the second electron transporting layer was changed as follows.
Device 3 of the invention: the dope amount of Li was 0.1% by mass thereof.
Device 5 of the invention: the dope amount of Li was 0.6% by mass thereof.
Device 6 of the invention: the dope amount of Li was 1 % by mass thereof.

4) Devices 7 to 10 of the invention
In the same way as in the formation of the device 1 of the invention, devices 7 to 10 of the invention were produced which each had the same composition as the device 1 of the invention except that Bathophenanthroline was used as a compound represented by formula (ET) in the second electron transporting layer instead of BCP, and the dope amount of Li relative to the amount of Bathophenanthroline was varied as follows.
Device 7 of the invention: the dope amount of Li was 0.1% by mass thereof.
Device of the invention: the dope amount of Li was 0.4% by mass thereof.
Device 9 of the invention: the dope amount of Li was 0.6% by mass thereof.
Device 10 of the invention: the dope amount of Li was 1% by mass thereof.

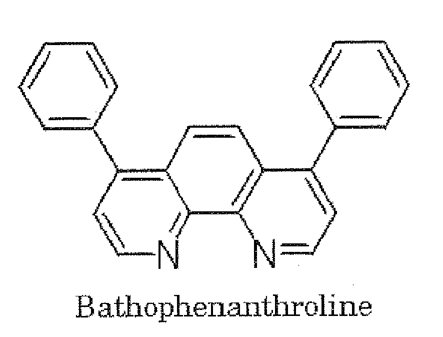

5) Devices 11 to 14 of the invention
In the same way as in the formation of the device I of the invention, devices 11 to 14 of the invention were produced which each had the same composition as the device 1 of the invention except that BCP in the second electron transporting layer being doped with Cs, and the dope amount of Cs relative to the amount of BCP being changed as follows.
Device I of the invention: the dope amount of Cs was 0.1% by mass thereof.
Device 12 of the invention: the dope amount of Cs was 0.4% by mass thereof.
Device 13 of the invention: the dope amount of Cs was 0.6% by mass thereof.
Device 14 of the invention: the dope amount of Cs was 1 % by mass thereof.

6) Devices 15 to 18 of the invention
In the same way as in the formation of the device 2 of the invention, devices 15 to 18 of the invention were produced which each had the same composition as the device 2 of the invention except that BCP in the second electron transporting layer being doped with Cs, and the dope amount of Cs relative to the amount of BCP being varied as follows.
Device 15 of the invention: the dope amount of Cs was 0.1% by mass thereof.
Device 16 of the invention: the dope amount of Cs was 0.4% by mass thereof.
Device 17 of the invention: the dope amount of Cs was 0.6% by mass thereof.
Device 18 of the invention: the dope amount of Cs was 1% by mass thereof.

7) Device 19 of the invention
In the same way as in the formation of the device 1. of the invention, a device 19 of the invention was produced which had the same composition as the device 1 of the invention except that BCP in the second electron transporting layer being doped with Cs₂O₃ for giving a dopant-proportion of 0.4% by mass of BCP.

8) Device 20 of the invention
In the same way as in the formation of the device 1 of the invention, a device 20 of the invention was produced which had the same composition as the device 1 of the invention except that BCP in the second electron transporting layer being doped with Ca for giving a dopant-proportion of 0.4% by mass of BCP.

9) Device 21 of the invention
In the same way as in the formation of the device 1 of the invention, a device 21 of the invention was produced which had the same composition as the device 1 of the invention except that BCP in the second electron transporting layer being doped with CaF₂ for giving a dopant-proportion of 0.4% by mass of BCP.

10) Device 22 of the invention
In the same way as in the preparation of the device 1 of the invention, a device 22 of the invention was produced except that the exemplified compound 26 was used instead of Pt-1. The resultant device was evaluated in the same manner as in the device 1 of the invention.

11) Device 23 of the invention
In the same way as in the preparation of the device 1 of the invention, a device 23 of the invention was produced except that the exemplified compound 29 was used instead of Pt-1. The resultant device was evaluated in the same manner as in the device 1 of the invention.

### 12) Production of comparative devices

### <Comparative device 1>

In the same way as in the formation of the device 1 of the invention, a comparative device 1 was produced which had the same composition as the device 1 of the invention except that the second electron transporting layer being changed to a layer made only of BCP.

### <Comparative device 2>

In the same way as in the formation of the device 2 of the invention, a comparative device 2 was produced which had the same composition as the device 2 of the invention except that the second electron transporting layer being changed to a layer made only of BCP.

### <Comparative device 3>

In the same way as in the formation of the device 1 of the invention, a comparative device 3 was produced which had the same composition as the device 1 of the invention except that the luminescent layer being changed to the following:
luminescent layer: mCP as a host material, and
Ir(ppy)₃(tris(2-phenylpyridine)iridium) as a luminescent material were used to be co-evaporated and deposited to set the ratio by mass of mCP to Ir(ppy)₃ to 85/15.

<Comparative device 4>
In the same way as in the formation of the device 2 of the invention, a comparative device 4 was produced which had the same composition as the device 2 of the invention except that the luminescent layer being changed to the following:
luminescent layer: the hole transporting host material A as a host material, and Ir(ppy)₃ as a luminescent material were used to be co-evaporated and deposited to set the ratio by mass of the hole transporting host material A to Ir(ppy)₃ to 85/15.

Comparative device 5>
In the same way as in the formation of the comparative device 3, a comparative device 5 was produced which had the same composition as the comparative device 3 except that the material with which the second electron transporting layer being doped from Li to Cs.

### <Comparative device 6>

In the same way as in the formation of the comparative device 4, a comparative device 6 was produced which had the same composition as the comparative device 4 except that the material with which the second electron transporting layer being doped from Li to Cs₂CO₃.

Comparative example 7>
In the same way as in the formation of the device 1 of the invention, a comparative device 7 was produced which had the same composition as the device 1 of the invention except that the second electron transporting layer being changed into the following:
second electron transporting layer: instead of BCP, tris(8-hydroxyqinolinato)aluminum (abbreviated to Alq), and Li were co-evaporated and deposited to set the dope amount of Li to 0.4% by mass af Alq. The thickness was 20 nm.

### <Evaluation of performances of the organic EL devices>

### 1) External quantum efficiency

A device (trade name: SOURCE MEASURE UNIT 2400, manufactured by Toyo Corp.) was used to apply a DC voltage to each of the devices, thereby causing the device to emit light. The luminance thereof was measured with a luminance meter (trade name: BM-8, manufactured by Topcon Corp.). The emission spectrum and the emission wavelength were measured with a spectrum analyzer (trade name: PMA-11, manufactured by Hamamatsu Photonics K.K.). On the basis of these values, the external quantum efficiency at a luminance of 1000 cd/m² was calculated out by a luminance converting method.

2) Driving voltage
The device (trade name: SOURCE MEASURE UNIT 2400, manufactured by Toyo Corp.) was used to apply a DC voltage to each of the devices, thereby causing the device to emit light. The voltage generated when the value of a current caused to flow into the device reached to 10 mA/cm² was measured as the driving voltage of the device.

### 3) Driving endurance: luminance half-value period

A DC voltage was applied to each of the devices so as to give a luminance of 1000 cd/m², and continuously the device was driven. A measurement was made about the period up to the time when the luminance reached to 500 cd/m². This luminance half-value period was regarded as an index for the driving endurance of the device.

The resultant results are shown in Table 1.

**[Table 1]**

| Device Nos. | External quantum efficiency | Driving voltage | Driving endurance |
|---|---|---|---|
| | (value relative to the value of comparative device 1 which was regarded as 1.00) | (value relative to the value of comparative device 1 which was regarded as 1.00) | (Value relative to the value of comparative device 1 which was regarded as 1.00) |
| Device 1 of the invention | 1.60 | 0.55 | 1.90 |
| Device 2 of the invention | 1.50 | 0.75 | 2.00 |
| Device 3 of the invention | 1.30 | 0.70 | 1.80 |
| Device 5 of the invention | 1.30 | 0.75 | 1.70 |
| Device 6 of the invention | 1.20 | 0.80 | 1.30 |
| Device 7 of the invention | 1.40 | 0.75 | 1.55 |
| Device 8 of the invention | 1.50 | 0.70 | 1.65 |
| Device 9 of the invention | 1.20 | 0.85 | 1.25 |
| Device 10 of the invention | 1.10 | 0.80 | 1.20 |
| Device 11 of the invention | 1.40 | 0.70 | 1.90 |
| Device 12 of the invention | 1.65 | 0.65 | 2.10 |
| Device 13 of the invention | 1.40 | 0.80 | 1.75 |
| Device 14 of the invention | 1.25 | 0.85 | 1.40 |
| Device 15 of the invention | 1.30 | 0.75 | 1.60 |
| Device 16 of the invention | 1.55 | 0.72 | 1.70 |
| Device 17 of theinvention | 1.22 | 0.80 | 1.30 |
| Device 18 of the invention | 1.18 | 0.82 | 1.10 |
| Device 19 of the invention | 1.35 | 0.80 | 1.60 |
| Device 20 of the invention | 1.27 | 0.88 | 1.40 |
| Device 21 of the invention | 1.20 | 0.91 | 1.30 |
| Device 22 of the invention | 1.40 | 0.65 | 1.50 |
| Device 23 of the invention | 1.35 | 0.60 | 1.70 |
| Comparative device 1 | 1.00 | 1.00 | 1.00 |
| Comparative device 2 | 0.90 | 1.10 | 1.20 |
| Comparative device 3 | 0.80 | 1.40 | 0.70 |
| Comparative device 4 | 0.85 | 1.30 | 0.85 |
| Comparative device 5 | 1.10 | 1.00 | 0.90 |
| Comparative device 6 | 1.10 | 0.95 | 0.95 |
| Comparative device 7 | 0.70 | 1.50 | 0.75 |

As is evident from the above-mentioned results, by doping a compound represented by formula (ET) with at least one selected from the group consisting of alkali metals, alkali metal salts, alkaline earth metals and alkaline earth metal salts, the EL device is improved in external quantum efficiency, driving voltage, and endurance. It is particularly effective to dope the compound with the dopant(s) to give a dopant-proportion of 0.4% by mass or 0.6 by mass of the compound.

All the contents of Japanese Patent Application No. 2008-298212 filed on November 21, 2008 are incorporated into the present specification by reference thereto.

## Claims

1. An organic electroluminescent device, comprising a pair of electrodes and an organic compound layer disposed between the pair of electrodes, the organic compound layer comprising at least a luminescent layer, a first electron transporting layer provided so as to contact a cathode-side surface of the luminescent layer, and a second electron transporting layer provided so as to contact a cathode-side surface of the first electron transporting layer, wherein the luminescent layer comprises at least a luminescent material represented by following formula (1) and a hole transporting host material, and the second electron transporting layer comprises at least an electron transporting material represented by following formula (ET) and at least one selected from the group consisting of alkali metals, alkali metal salts, alkaline earth metals and alkaline earth metal salts: 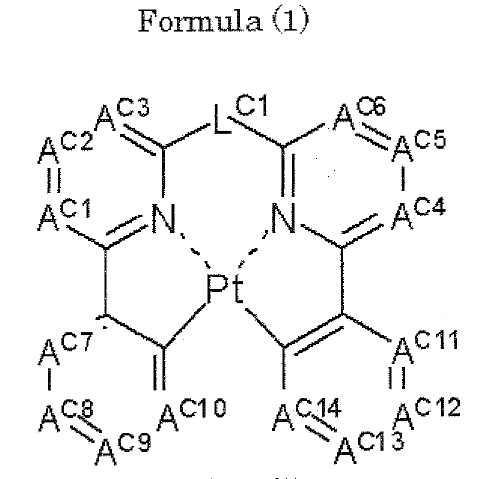 wherein, in formula (1), A^{C1} to A^{C14} each independently represents C-R or N, wherein R represents a hydrogen atom or a substituent; and L^{C1} represents a single bond or a bivalent linking group; and 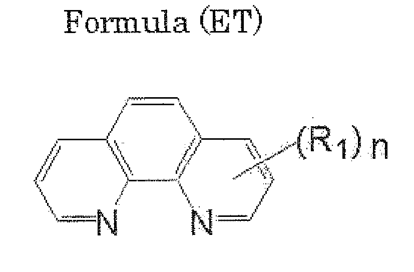 wherein, in formula (ET), R₁ each represents a hydrogen atom, or a substituent selected from the group consisting of alkyl, groups having to 10 carbon atoms and substituted or unsubstituted aryl groups having 6 to 30 carbon atoms; and n represents an integer of 0 to 8 provided that when n is an integer of 2 or more, the R₁'s may be the same as or different from each other.

2. The organic electroluminescent device of claim 1, wherein the compound represented by formula (1) is a compound represented by following formula (2): 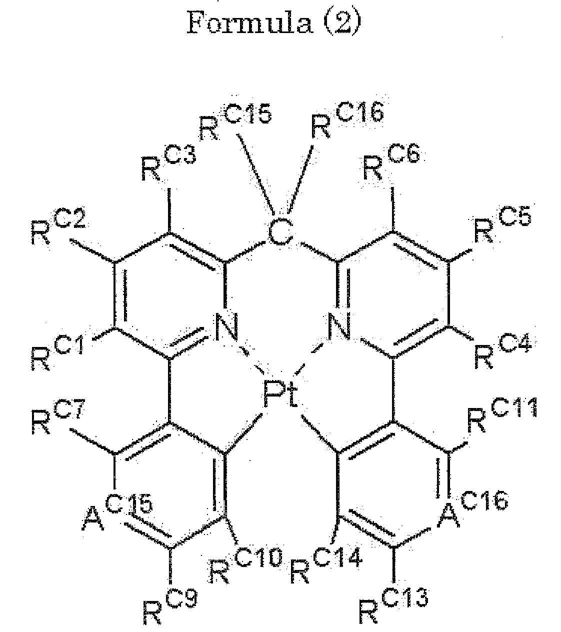 wherein, in formula (2), A^{C15} and A^{C16} each independently represents C-R or N, wherein R represents a hydrogen atom or a substituent; and R^{C1} to R^{C16} each represent a hydrogen atom or a substituent.

3. The organic electroluminescent device of claim 1, wherein the at least one selected from the group consisting of the alkali metals, alkali metal salts, alkaline earth metals, and alkaline earth metal salts is selected from Li, Cs, Ca, LiF, NaF, KF, RbF, CsF, MgF₂, CaF₂, SrF₂, BaF₂, LiCl, NaCl, KCl, RbCl, CsCl, MgCl₂, CaCl₂, SrCl₂, BaCl₂, LiHCO₃, NaHCO₃ KHCO₃, RbHCO₃, CsHCO₃, Li₂CO₃, Na₂CO₃, K₂CO₃. Rb₂CO₃, Cs₂CO₃, MgCO₃, CaCO₃, SrCO₃, and BaCO₃.

4. The organic electroluminescent device of claim 1, wherein in the second electron transporting layer, a ratio by mass of a total of the selected alkali metal(s), alkali metal salt(s), alkaline earth metal(s) and alkaline earth metal salt(s) in the electron transporting material represented by formula (ET) is from 0.1 to 2.0% by mass.

5. The organic electroluminescent device of claim 1, wherein the hole transporting host material is a carbazole derivative.

## Amended claims

### Amended claims under Art. 19.1 PCT

1. (Amended) An organic electroluminescent device, comprising a pair of electrodes and an organic compound layer disposed between the pair of electrodes, the organic compound layer comprising at least a luminescent layer, a first electron transporting layer provided so as to contact a cathode-side surface of the luminescent layer, and a second electron transporting layer provided so as to contact a cathode-side surface of the first electron transporting layer, wherein the luminescent layer comprises at least a luminescent material represented by following formula (2) and a hole transporting host material, and the second electron transporting layer comprises at least an electron transporting material represented by following formula (ET) and at least one selected from the group consisting of alkali metals, alkali metal salts, alkaline earth metals and alkaline earth metal salts: 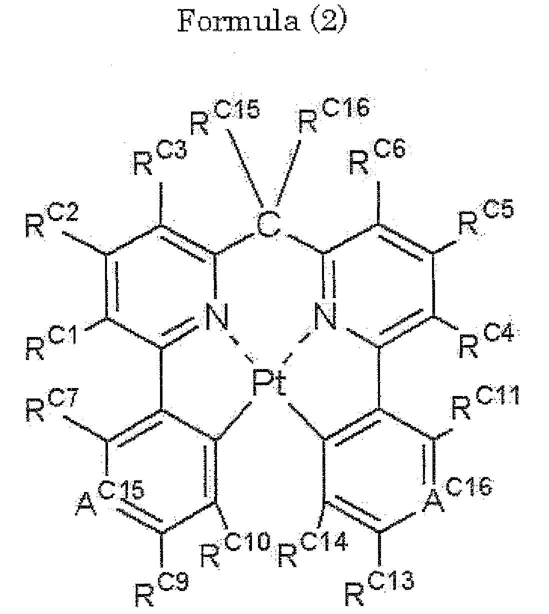 wherein, in formula (2), A^{C15} and A^{C16} each independently represents C-R or N, wherein R represents a hydrogen atom or a substituent; and R^{C1} to R^{C16} each represent a hydrogen atom or a substituent.; and 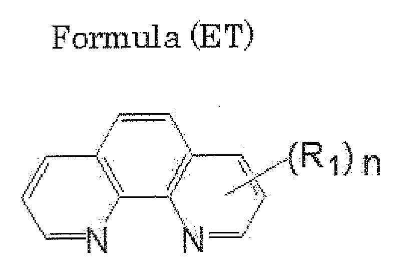 wherein, in formula (ET), R₁ each represents a hydrogen atom, or a substituent selected from the group consisting of alkyl groups having 1 to 10 carbon atoms and substituted or unsubstituted aryl groups having 6 to 30 carbon atoms; and n represents an integer of 0 to 8 provided that when n is an integer of 2 or more, the R₁'s may be the same as or different from each other.

2. (Deleted)

3. The organic electroluminescent device of claim 1, wherein the at least one selected from the group consisting of the alkali metals, alkali metal salts, alkaline earth metals, and alkaline earth metal salts is selected from Li, Cs, Ca, LiF, NaF, KF, RbF, CsF, MgF₂, CaF₂, SrF₂, BaF₂, LiCl, NaCl, KCl, RbCl, CsCl, MgCl₂, CaCl₂, SrCl₂, BaCl₂, LiHCO₃, NaHCO₃, KHCO₃, RbHCO₃, CsHCO₃, Li₂CO₃, Na₂CO₃, K₂CO₃, Rb₂CO₃, Cs₂CO₃, MgCO₃, CaCO₃, SrCO₃, and BaCO₃.

4. The organic electroluminescent device of claim 1, wherein in the second electron transporting layer, a ratio by mass of a total of the selected alkali metal(s), alkali metal salt(s), alkaline earth metal(s) and alkaline earth metal salt(s) in the electron transporting material represented by formula (ET) is from 0.1 to 2.0% by mass.

5. The organic electroluminescent device of claim I, wherein the hole transporting host material is a carbazole derivative.
